# EUROPEAN PATENT APPLICATION

(11) **EP 4 215 522 A1**
(43) Date of publication of application: **26.07.2023**
(21) Application number: 23152590.8
(22) Date of filing: 20.01.2023
(51) Int. Cl.: C07D 311/30, A61P 31/14, A61K 31/352

(54) **DERIVATIVES OF 4-CHROMONE AS INHIBITORS OF THE RDRP POLYMERASE OF SARS-COV-2 VIRUS AND METHOD FOR THE PREPARATION THEREOF**

(30) Priority: 20.01.2022 IT 202200000920
(71) Applicant: Fondazione Istituto Italiano di Tecnologia, 16163 Genova (IT)
(72) Inventor: DE VIVO, Marco, 16163 Genova (IT); BRINDANI, Nicoletta, 16163 Genova (IT); MENICHETTI, Andrea, 16163 Genova (IT)
(74) Representative: Croce, Valeria

(57) **Abstract**

The present invention relates to 4-chromone derivative compounds of general formula (I) and the medical use thereof as SARS-CoV-2 virus RdRp polymerase inhibitors.

## Description

### Technical field of the invention

The present invention relates to the medical field, and in particular for treating diseases caused by RNA viruses, in particular by SARS-CoV-2.

The health emergency due to the outbreak of acute respiratory syndrome coronavirus 2 (SARS-CoV-2) has not only affected the world population in terms of lost lives, but also in terms of economic impact on the national healthcare budgets.

The current situation has prompted the scientific community to search a variety of strategies against the spread of the coronavirus, including widespread immunization, local epidemiological monitoring and the use of drugs (e.g., remdesivir) already known for other applications.

Critical issues have emerged regarding the suspected onset of adverse effects, the great variability of the individual response to these treatments and, above all, the emergence of uncontrollable mutant strains resistant to drugs, in particular of the class of nucleotide analogues, it remaining urgent to find an effective treatment.

SARS-CoV-2 is a single-stranded positive RNA virus, which infects the renin-angiotensin-aldosterone system causing a vasodilation effect and the replication thereof requires a complex mechanism in which several viral and host factors and proteins are involved, including RNA-dependent RNA polymerase (RdRp).

In recent years, RdRp has emerged as an attractive target for the development of new antiviral drugs due to the high conservation thereof among distant evolutionary RNA viruses, the absence of RdRp homologues in mammalian cells, the extensive knowledge on the structure and functions of RdRp and the easy development and consequent availability of biochemical assays for the rapid screening of large compound libraries (Piccarizzi/Mori, Molecules 2020) .

The enzyme RdRp catalyzes the synthesis of a forming RNA strand, adding ribonucleotide units to the 3'-hydroxyl terminal, building the RNA molecule in the 5'-3' direction. To carry out the polymerase activity, RdRp requires an RNA template, ribonucleotide 5' triphosphate (ATP, GTP, UTP and CTP) as precursors of the forming RNA nucleotide units and two magnesium ions (Mg²⁺) within the active site which catalyze the formation of the phosphodiester bond.

The inhibitory action towards RdRp prevents the virus from proliferating, thus also blocking the resulting disease.

Despite the enormous effort of the scientific community to find an effective drug against SARS-CoV-2, currently a single RNA-dependent small molecule RNA polymerase inhibitor (RdRp), remdesivir, has been approved by the U.S. Food and Drug Administration (FDA) for the treatment of SARS-CoV-2 syndrome.

The use of remdesivir for the treatment against SARS-CoV-2 is part of the so-called "repurposing" strategy, which investigates the possible use of already known drugs for treating other diseases.

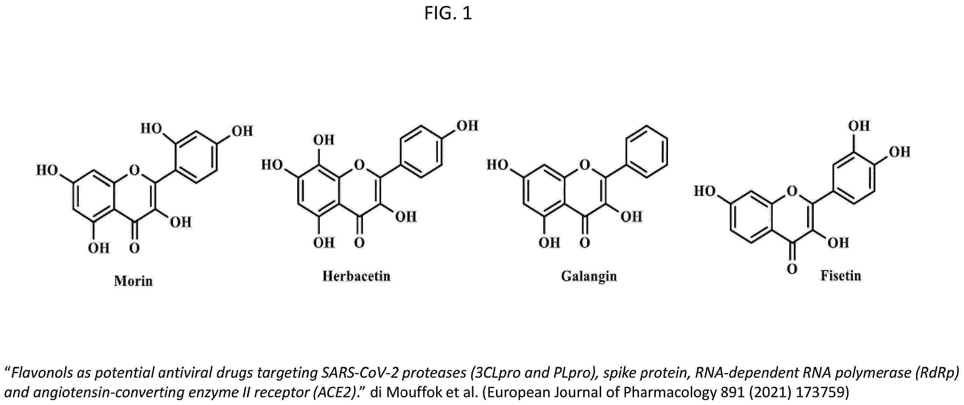

Prior art document *"Flavonols as potential antiviral drugs targeting SARS-CoV-2 proteases (3CLpro and PLpro), spike protein,* RNA-dependent RNA polymerase (RdRp) and angiotensin-converting enzyme II receptor (ACE2)." to Mouffouk et al. (European Journal of Pharmacology 891 (2021) 173759) describes the antiviral activity of natural compounds of plant origin, in particular of flavonols. Such compounds are shown in Figure 1.

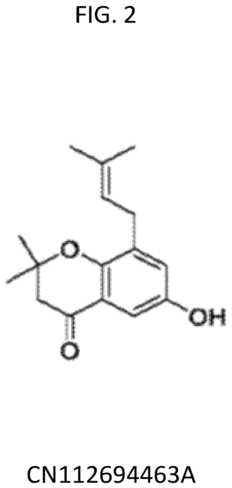

The Chinese patent application CN112694463A (Institute of medicinal biotechnology Chinese academy of medical sciences) describes compounds having anti-coronavirus activities characterized by the chroman-4-one structure shown in Figure 2. The mechanism used by such compounds to exert antiviral activity is not described.

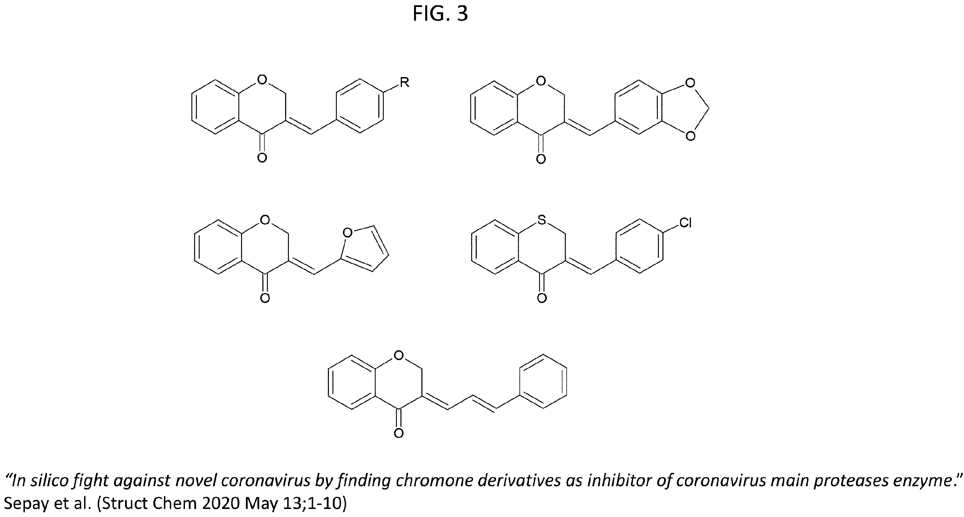

The prior art document *"*In silico fight against novel coronavirus by finding chromone derivatives as inhibitor of coronavirus main proteases enzyme." to Sepay et al. (Struct Chem 2020 May 13;1-10) describes naturally occurring compounds characterized by the structures shown in Figure 3 capable of blocking the main protease enzyme of SARS-CoV-2 (SARS-CoV-2 Mpro).

### Summary of the invention

The inventors of the present patent application have surprisingly found that the compounds characterized by the general formula (I) are capable of inhibiting the activity of the RdRp polymerase, which is essential for the virus replication in the host cells, and which can thus be usefully employed for treating viral infections.

Such an activity is completely unexpected based on the structure of similar compounds already known to have antiviral activity.

### Object of the invention

In a first object, the present patent application describes compounds for medical use.

According to a particular aspect, such compounds are described for medical use for treating diseases caused by RNA viruses.

According to an alternative aspect, such compounds are described for the prevention of diseases caused by RNA viruses.

According to a preferred aspect such diseases are caused by RNA viruses of the group comprising: SARS-CoV-2 virus, Hepatitis C virus (HCV), Ebola virus, Zika virus and further viruses: Severe acute respiratory syndrome coronavirus (SARS), Middle east respiratory syndrome coronavirus (MERS), influenza, Orthomyxoviruses, Paramyxoviruses, Hendra and Nipah viruses, Measles, Picornaviruses, Poliomyelitis ("Polio"), Hepatitis A virus (HAV), Rotavirus, Human immunodeficiency virus (HIV), adult Human T-cell lymphotropic virus type 1 (HTLV-1).

According to a particularly preferred aspect, such a disease is caused by the RNA virus represented by SARS-CoV-2.

In a second object, the present patent application describes pharmaceutical formulations comprising the compounds of the invention.

According to a particular aspect, such pharmaceutical formulations are described for medical use for treating diseases caused by RNA viruses.

According to a preferred aspect of the present invention such diseases are caused by RNA viruses of the group comprising: SARS-CoV-2 virus, Hepatitis C virus (HCV), Ebola virus, Zika virus and further viruses: Severe acute respiratory syndrome coronavirus (SARS), Middle east respiratory syndrome coronavirus (MERS), influenza, Orthomyxoviruses, Paramyxoviruses, Hendra and Nipah viruses, Measles, Picornaviruses, Poliomyelitis ("Polio"), Hepatitis A virus (HAV), Rotavirus, Human immunodeficiency virus (HIV), adult Human T-cell lymphotropic virus type 1 (HTLV-1).

According to a particularly preferred aspect, such a disease is caused by the RNA virus represented by SARS-CoV-2.

In a third object the present patent application describes compounds according to general formula (I) and general formulae (Ib) and (Ic).

In a fourth object, the present patent application describes a process for preparing the compounds of the invention.

In another object, the present patent application describes a method for treating or preventing diseases comprising the administration of a pharmaceutically effective amount of a compound according to the invention.

### Brief description of the drawings

Figures 1 to 3 show the structure of compounds known in the art.
Figures 4 to 10 show schemes 1 to 7, respectively, according to the present patent application.

### Detailed description of the invention

In a first object, the present patent application describes compounds having the general structures (I), (Ia), (Ib) and (Ic) and the formulae shown in Tables A, B and B' shown below for medical use.

According to a particular aspect, such compounds are described for medical use for treating diseases caused by RNA viruses.

According to a preferred aspect of the present invention such diseases are caused by RNA viruses of the group comprising: SARS-CoV-2 virus, *Hepatitis* C virus (HCV), Ebola virus, Zika virus and further viruses: Severe acute respiratory syndrome coronavirus (SARS), Middle east respiratory syndrome coronavirus (MERS), influenza, Orthomyxoviruses, Paramyxoviruses, Hendra and Nipah viruses, Measles, Picornaviruses, Poliomyelitis ("Polio"), Hepatitis A virus (HAV), Rotavirus, Human immunodeficiency virus (HIV), adult Human T-cell lymphotropic virus type 1 (HTLV-1).

According to a particularly preferred aspect, such a disease is caused by the RNA virus represented by SARS-CoV-2.

According to alternative aspects of the present invention, the compounds described are further intended for medical use in the prevention of diseases caused by RNA viruses.

According to a preferred aspect such diseases are caused by RNA viruses of the group comprising: SARS-CoV-2 virus, *Hepatitis* C virus (HCV), Ebola virus, Zika virus and further viruses: Severe acute respiratory syndrome coronavirus (SARS), Middle east respiratory syndrome coronavirus (MERS), influenza, Orthomyxoviruses, Paramyxoviruses, Hendra and Nipah viruses, Measles, Picornaviruses, Poliomyelitis ("Polio"), Hepatitis A virus (HAV), Rotavirus, Human immunodeficiency virus (HIV), adult Human T-cell lymphotropic virus type 1 (HTLV-1).

According to a particularly preferred aspect, such a disease is caused by the RNA virus represented by SARS-CoV-2.

For the purposes of the present invention, the medical use in the treatment and prevention described above concerns compounds according to general formula (I): 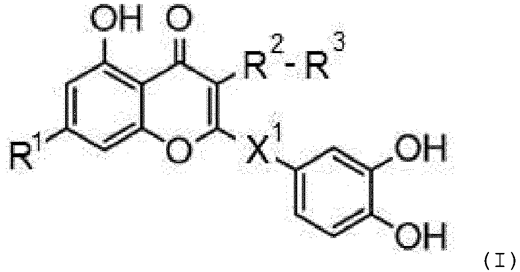 in which
R¹ can be hydrogen or -OH, -N(R₄)R₅, -NO₂, halogen, C₁₋₄ haloalkyl,
   where
R₄ and R₅ can be independently H, C₁₋₄ alkyl,
R² can be hydrogen, C₁₋₄ alkyl, C₁₋₄ haloalkyl, C₁₋₇ alkoxyalkyl and preferably C₁₋₄ alkoxyalkyl, or -O-, -NH-,
R³ can be H, -OH, or it can be C₁₋₄ alkyl, C₁₋₄ hydroxyalkyl, C₁₋₄ haloalkyl, C₁₋₄ aminoalkyl, C₁₋₄ alkoxyalkyl,
X¹ can be a single bond, X² or X³.

In a first embodiment, in the compounds of general formula (I) for medical use, R¹ is -OH and X¹ is a single bond and the compounds of the invention can thus have general formula (Ia):

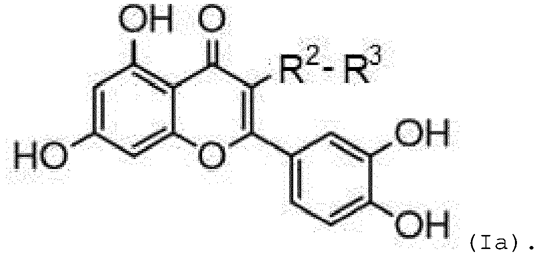

For the purposes of the present invention, in general formula (Ia) R² is -O- and R³ is C₁₋₄ alkyl or C₁₋₄ hydroxyalkyl.

In a second embodiment, in the compounds of general formula (I) for medical use, R¹ and R² are both hydrogen, X¹ is X² and the compounds of the invention can thus have general formula (Ib): 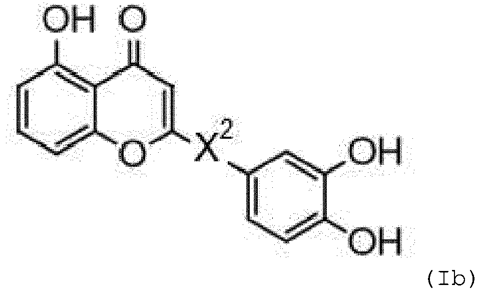
where X² can be -CH₂-, -CH₂CH₂-, -CH=CH-, -CHR⁴, -COR⁵, -NH, -O-, or -OR⁶, where
R⁴ can be C₁₋₄ alkyl, C₁₋₄ haloalkyl, C₁₋₄ alkoxyalkyl, C₁₋₄ aminoalkyl,
R⁵ can be -NH-, -O-, -NHR⁶-, -OR⁶-,
R⁶ can be C₂₋₄ alkyl.

For the purposes of the present invention, in general formula (Ib), X² is preferably selected from -CH₂-, -CH₂CH₂-, -CH=CH-, -CHR⁴, -COR⁵ and -OR⁶, where
R^{4,} R⁵ and R⁶ are as defined above.

In a third embodiment, in the compounds of general formula (I) for medical use, R¹ and R² are both hydrogen, X¹ is X³ and the compounds of the invention can thus have general formula (Ic): 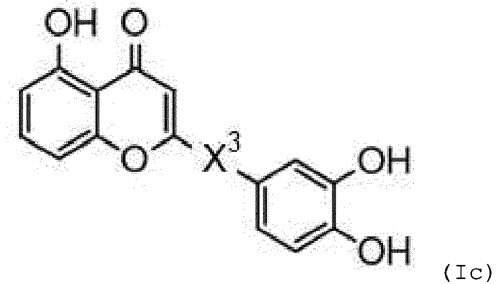
where X³ can be -C(O)X⁴ or -C(O)X⁴-(CH₂)₂₋₇-X⁵ and preferably - C(O)X⁴-(CH₂)₂₋₄-X⁵ where
X⁴ can be -NH, -O- or -O-C₂₋₄ alkyl,
X⁵ can be selected from: 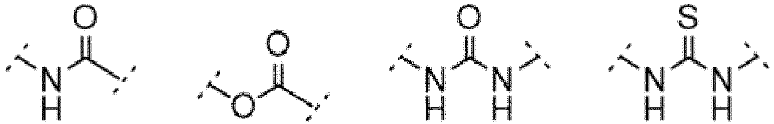 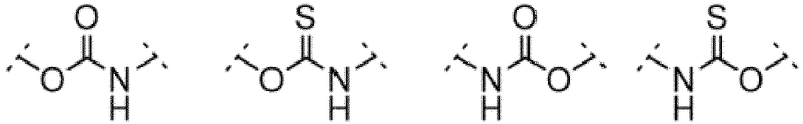 or
X⁵ can be -NH-R⁷, -OR⁷ or -OC₂₋₄ alkyl-R⁷ where
R⁷ can be -C(O), -C(O)NH or -C(S)NH, -C(O)O-, -C(S)O-.

According to an aspect of the present invention, the medical use of the compounds of the following Table A is particularly described:

| Ref | Formula | CAS |
|---|---|---|
| **(5a)** | 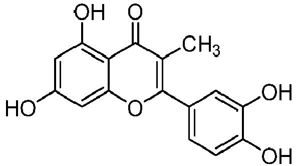 | 87339-67-1 |
| **(5b)** | 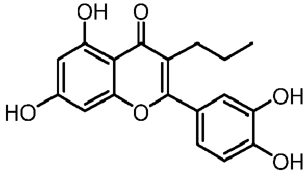 | |
| **(5c)** | 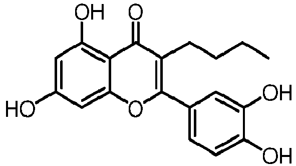 | 1052191-74-8 |
| **(9a)** | 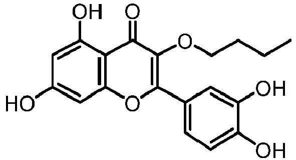 | 1453201-09-6 |
| **(9b)** | 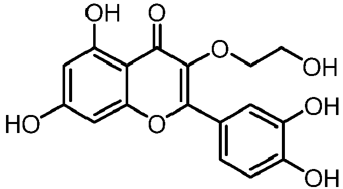 | 1428945-02-1 |
| **(9c)** | 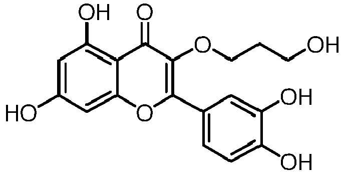 | 2832051-00-8 |
| **(13a)** | 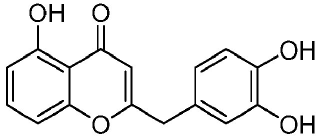 | |
| **(13b)** | 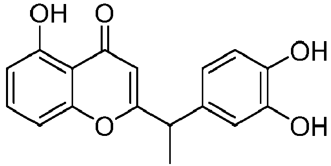 | |
| **(13c)** | 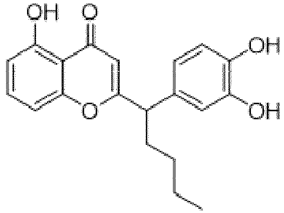 | |
| **(13d)** | 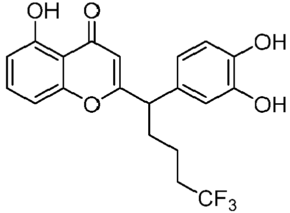 | |
| **(18)** | 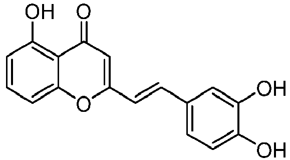 | 350855-59-3 |
| **(20)** | 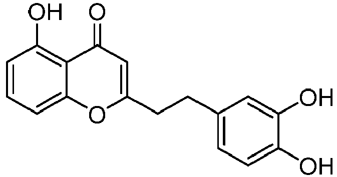 | |
| **(24a)** | 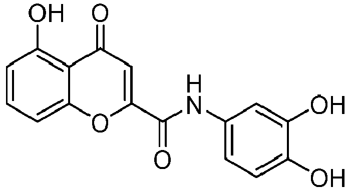 | |
| **(24b)** | 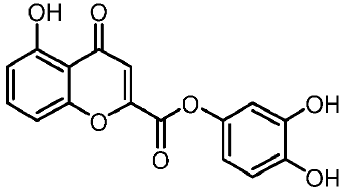 | |
| **(24c)** | 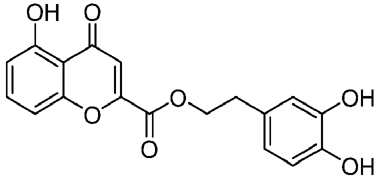 | |
| **(28)** | 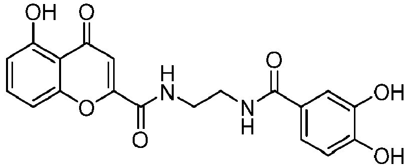 | |
| **(31)** | 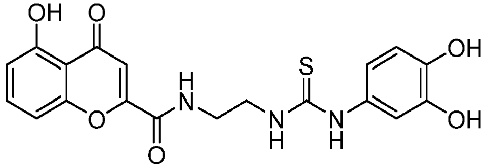 | |

In a preferred aspect of the present invention, the medical use of the compounds of the following Table B is described:

| Ref | Formula | CAS |
|---|---|---|
| **(5a)** | 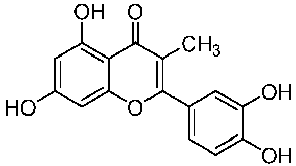 | 87339-67-1 |
| **(5b)** | 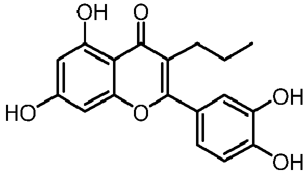 | |
| **(5c)** | 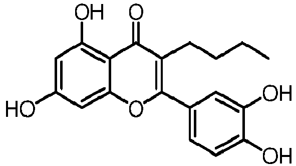 | 1052191-74-8 |
| **(9a)** | 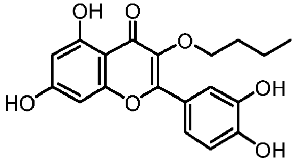 | 1453201-09-6 |
| **(9b)** | 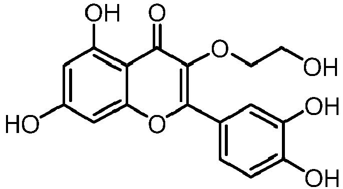 | 1428945-02-1 |
| **(9c)** | 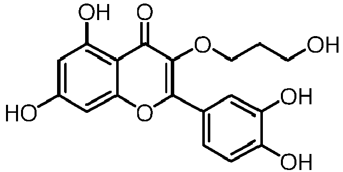 | 2832051-00-8 |
| **(13a)** | 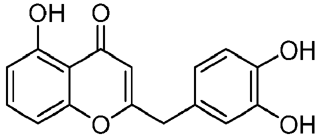 | |
| **(13b)** | 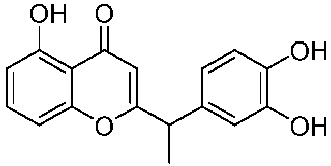 | |
| **(18)** | 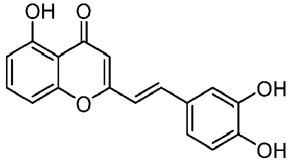 | 350855-59-3 |
| **(20)** | 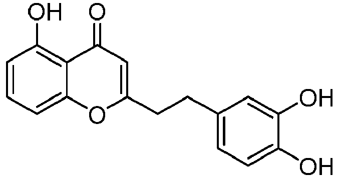 | |
| **(24a)** | 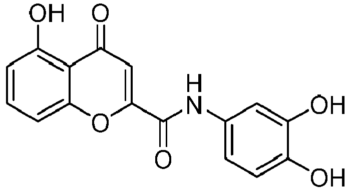 | |
| **(24b)** | 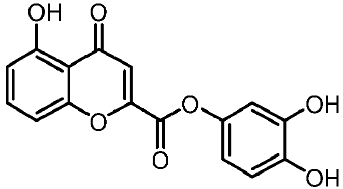 | |
| **(24c)** | 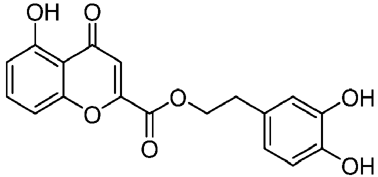 | |
| **(28)** | 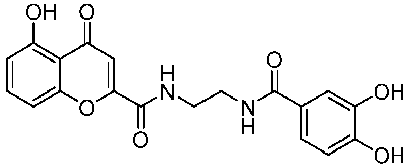 | |
| **(31)** | 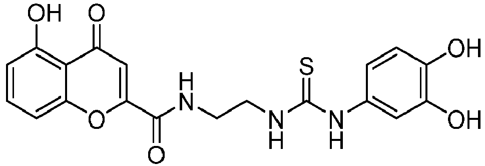 | |

In an even more preferred aspect of the present invention, the medical use of the compounds of the following Table B' is described:

| Ref | Formula | CAS |
|---|---|---|
| **(9a)** | 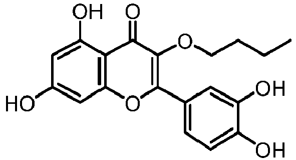 | 1453201-09-6 |
| **(9b)** | 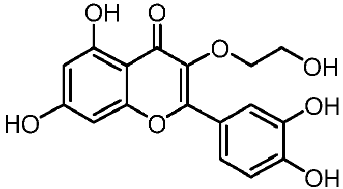 | 1428945-02-1 |
| **(9c)** | 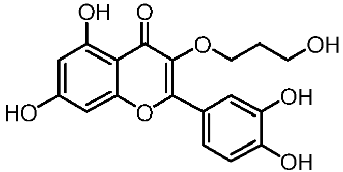 | 2832051-00-8 |
| **(13a)** | 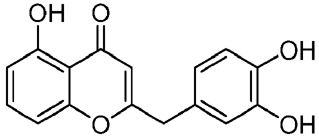 | |
| **(13b)** | 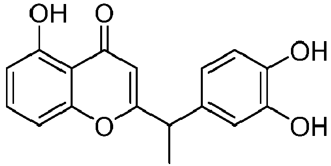 | |
| **(20)** | 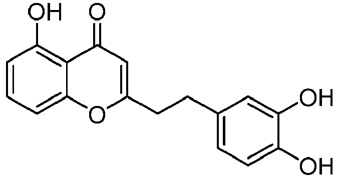 | |
| **(24a)** | 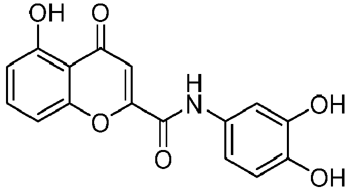 | |

In a second object, the present patent application describes pharmaceutical formulations comprising the compounds of the invention.

Such formulations can be administered orally, nasally, subcutaneously or intramuscularly.

In particular, such formulations comprise one or more pharmaceutically suitable excipients depending on the route of administration.

According to a particular aspect of the invention, the formulations shown above are also described for medical use for treating or preventing diseases caused by RNA viruses.

According to a preferred aspect such diseases are caused by RNA viruses of the group comprising: SARS-CoV-2 virus, *Hepatitis* C virus (HCV), Ebola virus, Zika virus and further viruses: Severe acute respiratory syndrome coronavirus (SARS), Middle east respiratory syndrome coronavirus (MERS), influenza, Orthomyxoviruses, Paramyxoviruses, Hendra and Nipah viruses, Measles, Picornaviruses, Poliomyelitis ("Polio"), Hepatitis A virus (HAV), Rotavirus, Human immunodeficiency virus (HIV), adult Human T-cell lymphotropic virus type 1 (HTLV-1).

According to a particularly preferred aspect, such a disease is caused by the RNA virus represented by SARS-CoV-2.

In a third object, the present patent application describes the compounds according to general formula (I): 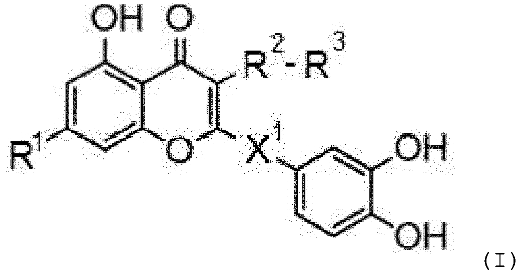 wherein
R¹ can be hydrogen or -OH, -N(R₄)R₅, -NO₂, halogen, C₁₋₄ haloalkyl, where
R₄ and R₅ can be independently H or C₁₋₄ alkyl,
R² can be hydrogen, C₁₋₄ alkyl, C₁₋₄ haloalkyl, C₁₋₇ alkoxyalkyl and preferably C₁₋₄ alkoxyalkyl, or -O-,-NH-,
R³ can be H, -OH, or it can be C₁₋₄ alkyl, C₁₋₄ hydroxyalkyl, C₁₋₄ haloalkyl, C₁₋₄ aminoalkyl, C₁₋₄ alkoxyalkyl,
X¹ can be X² or X³ as described hereinafter.

In a first embodiment, in the compounds of general formula (I), with the exclusion of the compound that is not the subject of the present invention, R¹ and R² are both hydrogen, X¹ is X² and the compounds of the invention can thus have general formula (Ib): 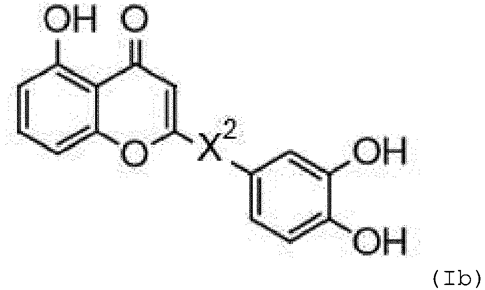 wherein
X² can be -CH₂-, -CH₂₋CH₂-, -CH=CH-, -CHR⁴, -COR⁵, -NH, -O- or -OR⁶, where
R⁴ can be C₁₋₄ alkyl, C₁₋₄ haloalkyl, C₁₋₄ alkoxyalkyl, C₁₋₄ aminoalkyl,
R⁵ can be -NH-, -O-, -NHR⁶-, -OR⁶-,
R⁶ can be C₂₋₄ alkyl.

For the purposes of the present invention, in general formula (Ib), X² is preferably selected from -CH₂-, -CH₂CH₂-, -CH=CH-, -CHR⁴, -COR⁵ and -OR⁶, where
R⁴, R⁵ and R⁶ are as defined above.

For the purposes of the present invention, in general formula (Ib), X² is preferably selected from -CH₂-, -CH₂CH₂-, -CH=CH-, -COR⁵ and -OR⁶, where
R⁵ and R⁶ are as defined above.

In the context of formula (Ib) shown above, the present invention does not relate to the following compound:

| Ref | Formula | CAS |
|---|---|---|
| **(18)** | 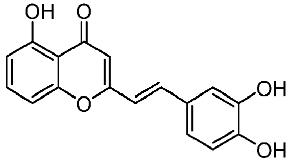 | 350855-59-3 |

In a second embodiment, in the compounds of general formula (I), with the exclusion of the compound that is not the subject of the present invention, and R¹ and R² are both hydrogen, X¹ is X³ and the compounds of the invention can thus have general formula (Ic): 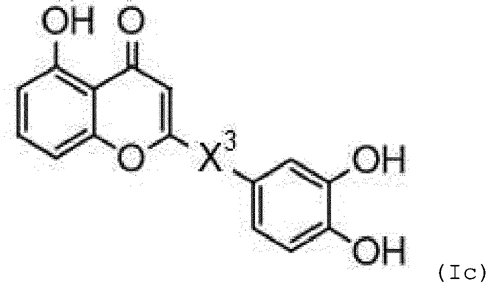 wherein
X³ can be -C(O)X⁴ or -C(O)X⁴-(CH₂)₂₋₇-X⁵ and preferably -C(O)X⁴-(CH₂)₂₋₄-X⁵ where
X⁴ can be -NH, -O- or -O-C₂₋₄ alkyl,
X⁵ can be selected from: 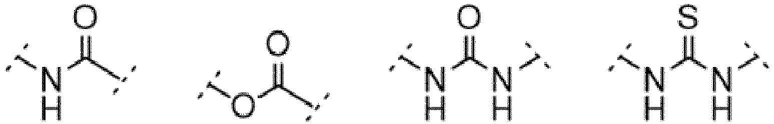 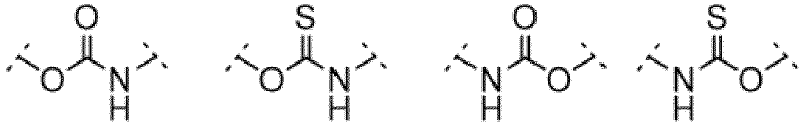 or
X⁵ can be -NH-R⁷, -OR⁷ or -OC₂₋₄ alkyl-R⁷ where
R⁷ can be -C(O), -C(O)NH or -C(S)NH, -C(O)O-, -C(S)O-.

Preferred compounds according to the present invention are the compounds having the formula shown in the following Table C:

| Ref | Formula |
|---|---|
| **(13c)** | 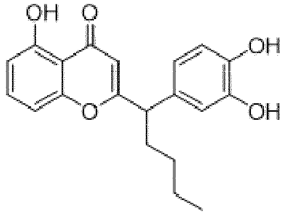 |
| **(13d)** | 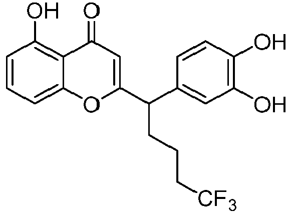 |
| **(20)** | 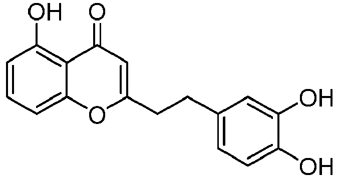 |
| **(24a)** | 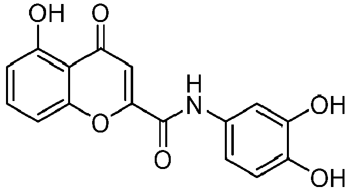 |
| **(24b)** | 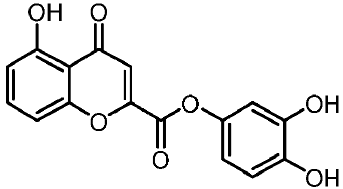 |
| **(24c)** | 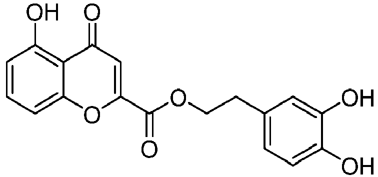 |
| **(28)** | 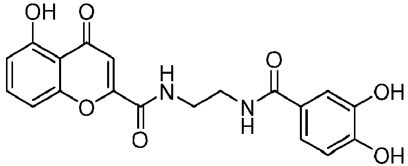 |
| **(31)** | 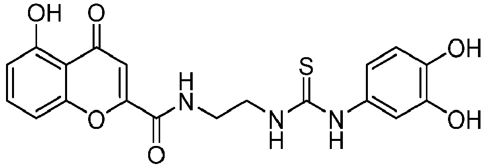 |

In accordance with a fourth object of the present patent application, a process for preparing the compounds of the invention is described.

In accordance with a first aspect, the compounds of general formula Ia are prepared, in which R² is different from oxygen.

In particular, such a synthesis comprises aldol condensation, oxidative cyclization and removal of the protective group.

The conditions of such steps are shown at the beginning of the experimental part (general procedures 1, 2 and 3).

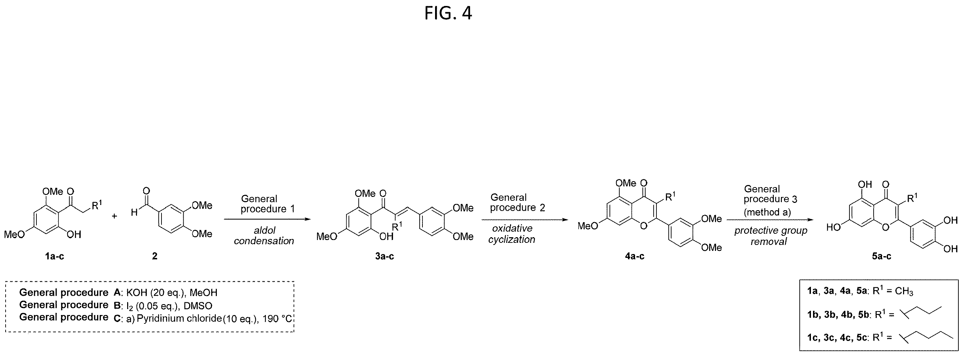

According to Scheme 1 shown in Figure 4, the compounds of formula **5a-c** are prepared, the synthesis of which is described in detail in the following examples.

In accordance with a second aspect, compounds of general formula Ia are prepared, in which R² is oxygen.

In particular, such a synthesis comprises an alkylation of a suitable 3-hydroxy-chromone and a benzyl deprotection.

The conditions of such steps are shown at the beginning of the experimental part (general procedures 4 and 5).

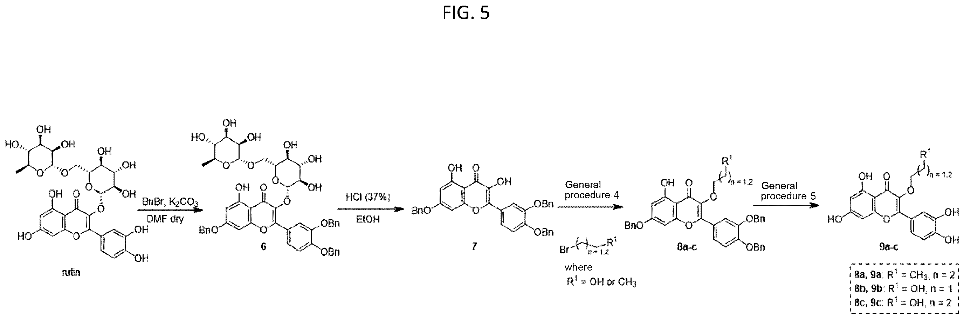

According to Scheme 2 shown in Figure 5, the compounds of formula **9a-c** are prepared, the synthesis of which is described in detail in the following examples.

In accordance with a third aspect, compounds of general formula Ib are prepared in which X² is -CH₂- or -CHR⁴.

In particular, such a synthesis comprises a Claisen condensation and a dehydrative cyclization, and a removal of the protective group.

In some cases, the Claisen condensation is preceded by an α-alkylation of the starting ester and subsequent hydrolysis.

The conditions of such steps are shown at the beginning of the experimental part (general procedures 6-9 and 3).

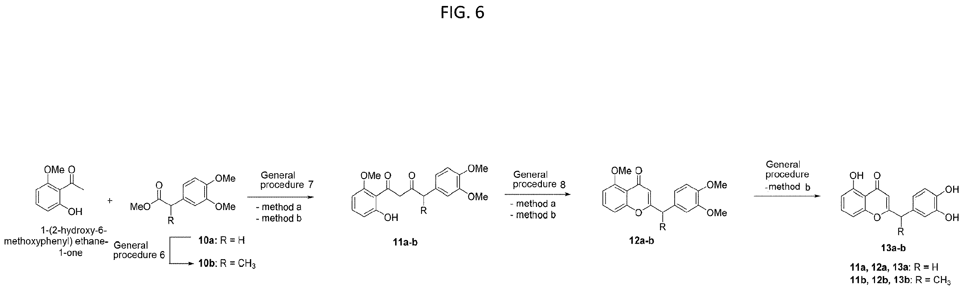

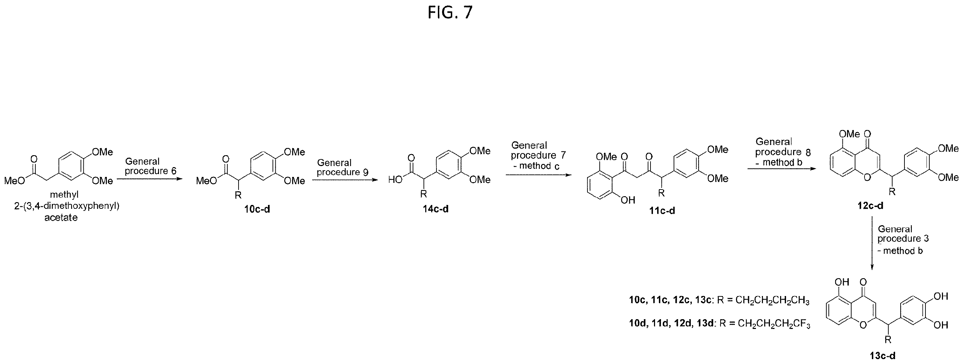

According to Scheme 3 and Scheme 4 shown in Figure 6 and Figure 7, respectively, the compounds of formula **13a-d** are prepared, the synthesis of which is described in detail in the following examples.

In accordance with a fourth aspect, compounds of general formula Ib are prepared in which X² is -CH₂₋CH₂- or -CH=HC-.

In particular, such a synthesis comprises a Claisen condensation, a dehydrative cyclization and a deprotection.

The conditions of such steps are shown at the beginning of the experimental part (general procedures 7, 8 and 3).

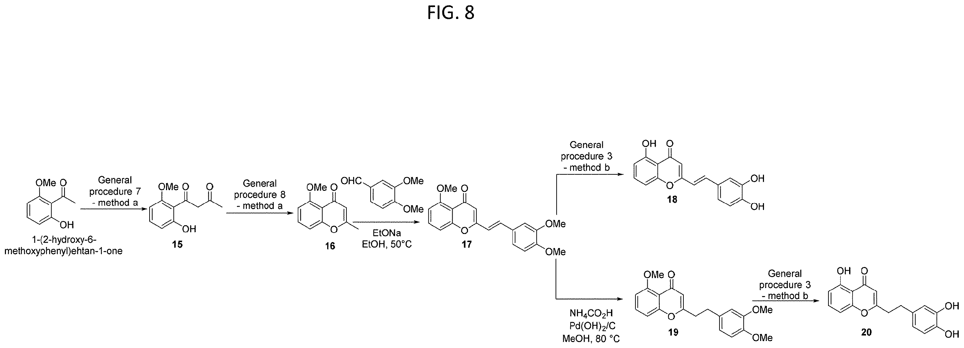

According to Scheme 5 shown in Figure 8, the compounds of formula **18** and **20** are prepared, the synthesis of which is described in detail in the following examples.

In accordance with a fifth aspect, compounds of general formula Ic are prepared in which X³ is -C(O)X⁴ in which X⁴ is -NH, -O- or -OC₂₋₄ alkyl.

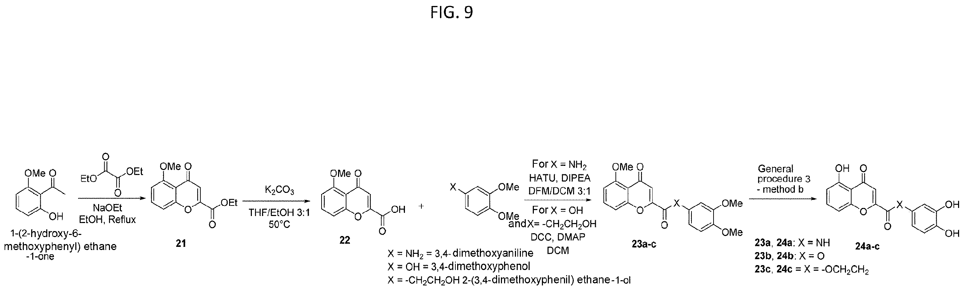

According to Scheme 6 shown in Figure 9 the compounds of **24a-c** are prepared, the synthesis of which is described in detail in the following examples.

In accordance with a sixth aspect, compounds of general formula Ic are prepared in which -C(O)X⁴-(CH₂)₂₋₇-X⁵.

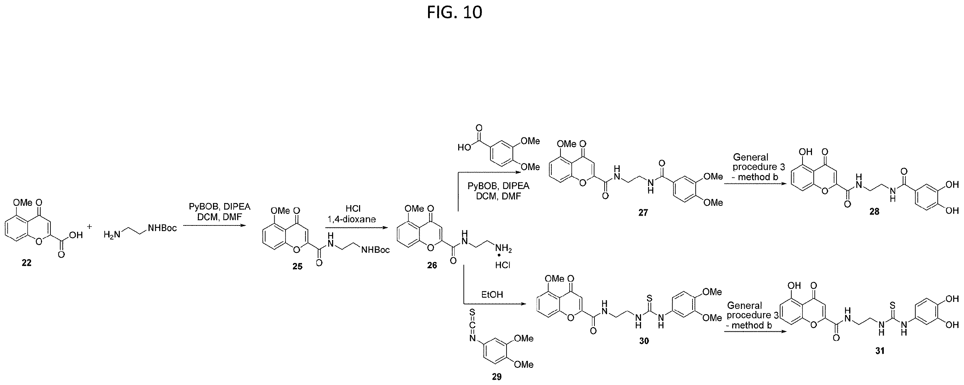

According to Scheme 7 shown in Figure 10 the compounds **28** and **31** are prepared, the synthesis of which is described in detail in the following examples.

In another object, the present patent application describes a method for treating diseases comprising administering a pharmaceutically effective amount of a compound or a pharmaceutical formulation according to the invention to a subject in need thereof.

For the purposes of the present invention, a subject in need thereof is a subject affected by a disease caused by RNA viruses.

According to a preferred aspect such diseases are caused by RNA viruses of the group comprising: SARS-CoV-2 virus, Hepatitis C virus (HCV), Ebola virus, Zika virus and further viruses: Severe acute respiratory syndrome coronavirus (SARS), Middle east respiratory syndrome coronavirus (MERS), influenza, Orthomyxoviruses, Paramyxoviruses, Hendra and Nipah viruses, Measles, Picornaviruses, Poliomyelitis ("Polio"), Hepatitis A virus (HAV), Rotavirus, Human immunodeficiency virus (HIV), adult Human T-cell lymphotropic virus type 1 (HTLV-1).

According to a particularly preferred aspect, such a disease is caused by the RNA virus represented by SARS-CoV-2.

According to alternative aspects of the present invention, the described method is further intended for the prevention of a disease caused by RNA viruses.

According to a preferred aspect such diseases are caused by RNA viruses of the group comprising: SARS-CoV-2 virus, Hepatitis C virus (HCV), Ebola virus, Zika virus and further viruses: Severe acute respiratory syndrome coronavirus (SARS), Middle east respiratory syndrome coronavirus (MERS), influenza, Orthomyxoviruses, Paramyxoviruses, Hendra and Nipah viruses, Measles, Picornaviruses, Poliomyelitis ("Polio"), Hepatitis A virus (HAV), Rotavirus, Human immunodeficiency virus (HIV), adult Human T-cell lymphotropic virus type 1 (HTLV-1).

According to a particularly preferred aspect, such a disease is caused by the RNA virus represented by SARS-CoV-2.

### Methods

All the commercially available reagents and solvents were used as purchased from vendors without further purification. The intermediates **1a** and **1b** can be synthesized according to the procedure shown in European Journal of Medicinal Chemistry 180 (2019) 350-366. The intermediate **1c** can be synthesized according to the procedure shown in the international patent application WO 2017/132928 A1. Anhydrous solvents were purchased from Sigma-Aldrich. Automated column chromatography purifications were done using a Teledyne ISCO apparatus (CombiFlash^{®} Rf) with pre-packed silica gel columns of different sizes (from 4 g up to 120 g) and mixtures of increasing polarity of cyclohexane and ethyl acetate (EtOAc) or dichloromethane (DCM) and methanol (MeOH). NMR experiments were run on a Bruker Avance III 400 system (400.13 MHz for 1H, and 100.62 MHz for 13C), equipped with a BBI probe and Z-gradients. Spectra were acquired at 300 K, using deuterated dimethylsulfoxide (DMSO-d₆)or deuterated chloroform (CDCl₃) as solvents. For ¹H-NMR, data are shown as follows: chemical shift, multiplicity (s= singlet, d= doublet, dd= double of doublets, ddd= doublet of doublet of doublets, t= triplet, td= triplet of doublets, q= quartet, p= quintet, m= multiplet), coupling constants (Hz) and integration. UPLC/MS analyses were run on a Waters ACQUITY UPLC/MS system consisting of a SQD (single quadrupole detector) mass spectrometer equipped with an electrospray ionization interface and a photodiode array detector. The PDA range was 210-400 nm. The analyses were carried out on a C18 ACQUITY UPLC BEH column (100x2.1 mmID, 1.7 um particle size) with a C18 VanGuard BEH pre-column (5x2.1 mmID, 1.7 um particle size) or on ACQUITY UPLC HSS T3 C₁₈ column combined again with a C18 VanGuard BEH pre-column (5x2.1 mmID, 1.7 um particle size) . The mobile phase was 10 mM NH₄OAc in H₂O at pH 5 adjusted with CH₃COOH (A) and 10 mM NH₄OAc in CH₃CN-H₂O (95:5) at pH 5.0 (B). Two types of gradients were applied depending on the analysis, gradient 1 (5% to 100 % mobile phase B in 3 min) or gradient 2 (50% to 100 % mobile phase B in 3 min). Alternatively, analyses were performed on ACQUITY UPLC HSS T3 C₁₈ column combined again with a VanGuard BEH C18 pre-column (5x2.1 mmID, 1.7 um particle size). In this case, the type of gradient applied is gradient 3 (mobile phase B from 0% to 100%B in 3.0 min). Electrospray ionization in positive and negative mode was applied. ESI was applied in positive and negative mode. All tested compounds showed ≥ 95% purity by UPLC/MS analysis.

### General procedures

**General procedure 1. Aldol condensation (Scheme 1,** **Figure 4****)** . The appropriate ketone of type **1** (1.0 eq.) and benzaldehyde of type **2** (1.1 eq) were added to a solution of potassium hydroxide (20.0 eq.) in MeOH (0.12 M). The reaction mixture was stirred at room temperature. After complete conversion of starting materials, the reaction mixture was acidified to pH=5 with 1 M HCl and extracted with EtOAc (3x5 mL). Combined organic layers were dried over magnesium sulfate, filtered and concentrated under vacuum. The product was purified by flash chromatography or trituration with EtOH giving the pure intermediate of type **3.**

**General procedure 2. Oxidative cyclization (Scheme 1,** **Figure 4****).** The appropriate chalcone of type 3 (1 eq.) was dissolved in anhydrous DMSO (0.3 M) and heated at 135°C under argon atmosphere. I₂ (0.05 eq.) was added and the reaction mixture was stirred until full conversion of the starting material. After reaction completion, the reaction mixture was cooled to room temperature and sodium thiosulfate 1 N was added to quench iodine. The crude product was filtered, washed with water and purified by flash chromatography giving the pure compound of type **4.**

### General procedure 3. Demethylation (Schemes 1, 3-7, Figures 4, 6-10) .

*Method A.* The appropriate compound of type **4** (1 eq.) was treated with pyridinium chloride (10 eq.) and the reaction mixture was heated at 190°C under argon atmosphere until total conversion of the starting material. After reaction completion, the reaction mixture was cooled down to room temperature and added with water. The crude product was filtered, washed with water and purified by silica. *Method B.* The appropriate compound of type **12, 17, 19, 23, 27, 30** (1 eq.) was dissolved in anhydrous DCM (0.05 M) and cooled to 0°C. A 1 M solution of boron tribromide in DCM (1.5 eq for each methoxy group) was added and the reaction mixture was allowed to warm to room temperature and stirred until complete conversion of the starting material under argon. After reaction completion, the reaction was quenched with MeOH and concentrated under reduced pressure. The crude product was redissolved in MeOH, concentrated under vacuum and purified by flash chromatography.

### General procedure 4. Alkylation of 3-hydroxy chromone (Scheme 2, Figure 5)

K₂CO₃ (3 eq.) and suitable alkyl bromide (1.5 eq.) were added to a solution of intermediate **7** (1 eq.) in DMF (0.06 M) and the reaction mixture was stirred under argon for three hours at room temperature. After reaction completion, the reaction mixture was poured into water and extracted with ethyl acetate (2x20 mL). The combined organic layer was divided, dried over magnesium sulfate, filtered and concentrated under vacuum, giving a crude product of the compound of type **8,** which was used for the next step without purification.

### General procedure 5. Benzyl deprotection (Scheme 2, Figure 5)

An appropriate benzylated compound was dissolved in a 1:1 mixture MeOH/DCM (0.04 M) under an argon atmosphere. Pd/C (20% w/w) triethylsilane (6 eq. for each benzyl group) were added to the solution. The reaction mixture was stirred at 40°C until complete conversion of the starting material. Then, the reaction mixture was filtered over a bed of celite and concentrated under vacuum. The crude product was raised in ethyl acetate and the organic phase was washed with water, dried over magnesium sulfate, filtered and concentrated under vacuum, and purified by silica.

### General procedure 6. Alpha alkylation of esters (Schemes 3-4, Figures 6-7)

The starting material methyl-2-(3,4-dimethoxyphenyl)acetate **10a** (1.0 eq) is dissolved in anhydrous THF (2 M) at -78°C in an inert argon atmosphere and LiHMDS or LDA base (1.5 eq) was then added to the mixture at the usual temperature. After 40 minutes of stirring to form the desired enolate, the corresponding alkyl iodide (2.0 eq) was added dropwise at -78°C and the mixture thus obtained was allowed to reach room temperature until the starting material had completely disappeared (another 1.5-3 base equivalents and 1 alkyl iodide equivalent were added when starting material was still present after one night) . At the end of the reaction, ice and then 2 M HCl was slowly added up to pH 6. The mixture was then extracted with AcOEt and the obtained organic phases were dried with Na₂CO₃, the solvent evaporated and the obtained crude was purified on silica to give compounds **10b-d.**

### General procedure 7. Claisen Condensation (Schemes 3-5 Figures 6-8)

*Method A.* A solution of appropriate ketone (1 eq.) in anhydrous THF (0.3 M) was added to a suspension of 60% NaH dispersion in mineral oil (4 eq.) in anhydrous THF (1.2 M) under argon, followed by the addition of an appropriate ester (2 eq.) at room temperature. The reaction mixture was then reflux-stirred under argon until complete consumption of the starting material. The reaction mixture was quenched by pouring into ice and further acidified up to pH 6 with acq. 2 M HCl and then extracted with EtOAc. The collected organic layers were dried over Na₂SO₄, filtered, and concentrated under vacuum. The crude product was used as such without further purification.

*Method B.* A solution of appropriate ketone (1.0 eq.) in anhydrous THF (0.12 M) was added to LDA (3.0 eq.) under an inert argon atmosphere at -78°C and the mixture was stirred for 30 minutes at the usual temperature, then another 30 minutes at 0°C to form the corresponding enolate. It was then heated again to -78°C in order to add the corresponding ester (1.0 eq). The addition was performed dropwise over about 15 minutes and the reaction mixture was allowed to slowly come to room temperature. After 24 h the reaction is stopped by slowly adding water and then 2 M HCl up to acidic pH. The crude mixture was extracted with AcOEt, the collected organic phases were dried with Na₂SO₄, the solvent evaporated and the crude material filtered on silica.

*Method C.* In a closed and septum-sealed vial, the reference carboxylic acid (1.0 eq) was solubilized in anhydrous DCM (0.25 M) and, under inert argon or nitrogen environment, SOCl₂ (10.0 eq) was added. The mixture thus obtained was reflux-heated for about one hour. Then the solvent and excess thionyl chloride were evaporated under vacuum in order to obtain the corresponding acid chloride. This is dissolved in the minimum possible amount of anhydrous THF and added dropwise to the preformed enolate solution using the methodology described in method b described above (1.0 eq ketone, 0.12 M anhydrous THF, LDA 3.0 eq). The reaction was stirred overnight and was then stopped by adding H₂O and 2 M HCl up to acidic pH. The crude mixture was extracted with AcOEt, the collected organic phases were dried with Na₂SO₄, the solvent evaporated and the crude material filtered on silica.

### General procedure 8. Dehydrating cyclization (Schemes 3-5 Figures 6-8)

Method A. An appropriate diketone derived from step 1 (1 eq.) was treated with an 8:1 mixture of MeOH/37% HCl (0.15 M). The reaction mixture was stirred at room temperature until complete consumption of the starting material. Then the solvent was removed under vacuum, the residue was raised with EtOAc and washed with NaHCOs saturated solution. The organic layer was divided, dried over Na₂SO₄, filtered and concentrated under vacuum. Purification by typical silica gave the pure desired product.

*Method B.* To a solution of the crude material of compounds **11 b-d** in CH₃COOH (0.12 M), 0.25 mL of conc. H₂SO₄ per 4.0 mmol of starting material were added and the obtained mixture was heated to 100°C. After disappearance of the starting material, the solvent was evaporated, H₂O and AcOEt were added to the obtained residue, the aqueous phase was extracted three times with AcOEt and the combined organic phases were dried with Na₂SO₄, the solvent evaporated and the obtained crude material was purified on silica.

### General procedure 9. Ester hydrolysis (Scheme 4, Figure 7)

The corresponding ester was dissolved in a 10:1 mixture of THF/H₂O (0.1 M) and LiOH (2.0 eq) was added to the reaction mixture, which was heated at 50°C for 16 h. After the disappearance of the starting material, the mixture was cooled to room temperature and 2 M HCl was slowly added up to acidic pH. The mixture thus obtained was extracted with AcOEt, the combined organic phases were dried with Na₂SO₄, the solvent evaporated and the obtained crude was cleaned with a filtration on silica.

### EXAMPLE 1

### Synthesis of 2-(3,4-dihydroxyphenyl)-5,7-dihydroxy-3-methyl-4H-chromen-4-one (compound 5a, Scheme 1).

### Step 1. (E)-3-(3,4-dimethoxyphenyl)-1-(2-hydroxy-4,6-dimethoxyphenyl)-2-methylprop-2-en-1-one (compound 3a, Scheme 1).

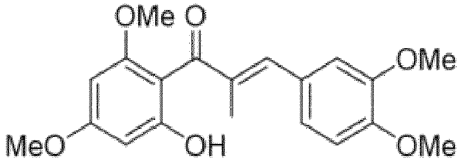

The title compound was synthesized following the general procedure 1 described above using 1-(2-hydroxy-4,6-dimethoxyphenyl)propane-1-one **1a** (100 mg, 0.48 mmol), 3,4-dimethoxybenzaldehyde **2** (87 mg, 0.53 mmol) and potassium hydroxide (539 mg, 9.6 mmol) in MeOH (5 mL). Purification by silica (elution by gradient from 100 to 80/20 cyclohexane/EtOAc).

**Characterization:** UPLC/MS Rt: 2.12 min (gradient 1), MS (ESI) m/z: 359.0 [M+H]⁺. [M+H]⁺ Calculated for C₂₀H₂₃O₆: 359.4. ¹H-NMR (400 MHz, DMSO-d₆): 7.12 (d, J = 1.5 Hz, 1H), 7.00 (s, 2H), 6.98 (s, 1H), 6.11 (d, J = 1.8, 1H), 6.07 (d, J = 1.9 Hz, 1H), 3.78 (s, 3H), 3.76 (s, 3H), 3.73 (s, 3H), 3.66 (s, 3H), 2.08 (s, 3H).

### Step 2. Synthesis of 2-(3,4-dimethoxyphenyl)-5,7-dimethoxy-3-methyl-4H-chromen-4-one (compound 4a, Scheme 1).

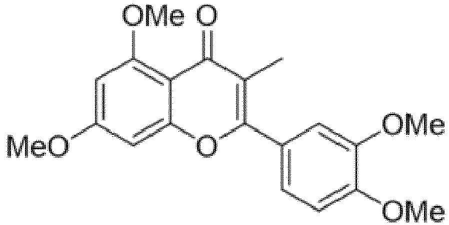

The title compound was synthesized following the general procedure 2 described above using intermediate **3a** (77 mg, 0.22 mmol) and I₂ (2.7 mg, 0.01 mmol) in DMSO (1 mL). Purification by silica (elution by gradient from 15/85 to 50/50 cyclohexane/EtOAc) gave the pure intermediate **4a** (59 mg, 77% yield).

**Characterization:** UPLC/MS Rt: 1.96 min (gradient 1), MS (ESI) m/z: 357.0 [M+H]⁺.[M+H]⁺ calculated for C₂₀H₂₁O₆: 357.1. ¹H-NMR (400 MHz, DMSO-d₆) δ 7.28 - 7.20 (m, 2H), 7.14 - 7.08 (m, 1H), 6.67 (d, J = 2.3 Hz, 1H), 6.48 (d, J = 2.3 Hz, 1H), 3.87 (s, 3H), 3.86 - 3.80 (m, 9H), 1.96 (s, 3H).

### Step 3. Synthesis of 2-(3,4-dihydroxyphenyl)-5,7-dihydroxy-3-methyl-4H-chromen-4-one (compound 5a, Scheme 1).

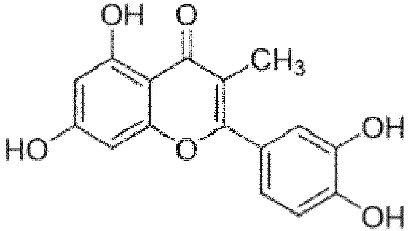

The title compound was synthesized following the general procedure 3 Method A described above using intermediate **4a** (59 mg, 0.16 mmol) and pyridinium chloride (185 mg, 1.6 mmol). Purification by silica (elution by gradient from 100 to 80:20 DCM/MeOH) gave the pure compound **5a** (31 mg, 74% yield).

**Characterization:** UPLC/MS Rt: 1.62 min (gradient 1), MS (ESI) m/z: 301.0 [M+H]⁺.[M+H]⁺ Calculated for C₁₆H₁₃O₆: 300.3. ¹H-NMR (400 MHz, DMSO-d₆) δ 13.07 (s, 1H), 7.08 (d, J = 2.1 Hz, 1H) 7.01 (dd, J = 8.3, 2.1 Hz, 1H), 6.88 (d, J = 8.3 Hz, 1H), 6.29 (bs, 1H), 6.15 (bs, 1H), 2.01 (s, 3H).

### EXAMPLE 2

### Synthesis of 2-(3,4-dihydroxyphenyl)-5,7-dihydroxy-3-propyl-4H-chromen-4-one (compound 5b, Scheme 1).

### Step 1. Synthesis of (E)-2-(3,4-dimethoxybenzylidene)-1-(2-hydroxy-4,6-dimethoxyphenyl)pentane-1-one (Intermediate 3b, Scheme 1)

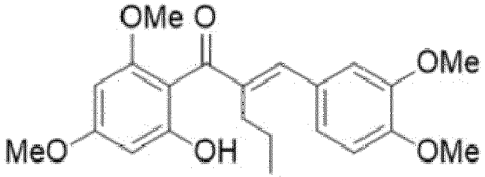

The title compound was synthesized following the general procedure 1 described above using 1-(2-hydroxy-4,6-dimethoxyphenyl)pentane-1-one **1b** (105 mg, 0.60 mmol), 3,4-dimethoxybenzaldehyde **2** (101 mg, 0.66 mmol) and potassium hydroxide (675 mg, 12 mmol) in MeOH (5 mL). Purification by silica (elution by gradient from 100 to 70/30 cyclohexane/EtOAc) gave the pure intermediate **3b** (116 mg, 50% yield).

**Characterization:** UPLC/MS Rt: 2.40 min (gradient 1), MS (ESI) m/z: 387.2 [M+H]⁺. [M+H]⁺ Calculated for C₂₂H₂₇O₆: 387.2. ¹H-NMR (400 MHz, DMSO-d₆) δ 9.70 (s, 1H), 7.04 (s, 1H), 7.01 (d, J = 8.1 Hz, 1H), 6.97 - 6.89 (m, 2H), 6.12 (d, J = 2.1 Hz, 1H), 6.07 (d, J = 2.1 Hz, 1H), 3.78 (s, 3H), 3.76 (s, 3H), 3.74 (s, 3H), 3.65 (s, 3H), 1.57 - 1.44 (m, 2H), 1.17 (t, J = 7.1 Hz, 2H), 0.96 (t, J = 7.3 Hz, 3H).

### Step 2. Synthesis of 2-(3,4-dimethoxyphenyl)-5,7-dimethoxy-3-propyl-4H-chromen-4-one (Intermediate 4b, Scheme 1).

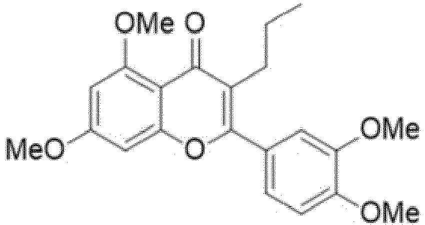

The title compound was synthesized following the general procedure 2 described above using intermediate **3b** (116 mg, 0.3 mmol) and I₂ (4 mg, 0.01 mmol) in DMSO (1 mL). Purification by silica (elution by gradient from 15/85 to 50/50 cyclohexane/EtOAc) gave the pure intermediate **4b** (54 mg, 50% yield).

**Characterization:** UPLC/MS Rt: 2.23 min (gradient 1), MS (ESI) m/z: 385.2 [M+H]⁺.[M+H]⁺ Calculated for C₂₂H₂₅O₆: 385.2. ¹H-NMR (400 MHz, DMSO-d₆) δ 7.20 - 7.15 (m, 2H), 7.12 (s, 1H), 6.64 (d, J = 2.3 Hz, 1H), 6.48 (d, J = 2.3 Hz, 1H), 3.85 (s, 3H), 3.84 (s, 3H), 3.83 (s, 3H), 3.81 (s, 3H), 2.38 - 2.29 (m, 2H), 1.53 - 1.39 (m, 2H), 0.82 (t, J = 7.3 Hz, 3H).

### Step 3. Synthesis of 2-(3,4-dihydroxyphenyl)-5,7-dihydroxy-3-propyl-4H-chromen-4-one (compound 5b, Scheme 1).

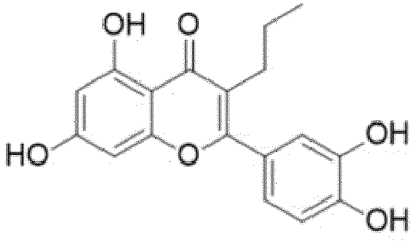

The title compound was synthesized following the general procedure C Method A described above using intermediate 1b (54 mg, 0.14 mmol) and pyridinium chloride (162 mg, 1.4 mmol). Purification by silica (elution by gradient from 100 to 94:6 DCM/MeOH) gave the pure compound **1** (35 mg, 76% yield).

**Characterization:** UPLC/MS Rt: 1.93 min (gradient 1), MS (ESI) m/z: 329.3 [M+H]⁺.[M+H]⁺ Calculated for C₁₈H₁₇O₆: 329.1. ¹H-NMR (400 MHz, DMSO-d₆) δ 13.08 (s, 1H), 7.01 (d, J = 2.1 Hz, 1H) 6.93 (dd, J = 8.2, 2.1 Hz, 1H), 6.88 (d, J = 8.2 Hz, 1H), 6.28 (d, J = 2.1 Hz, 1H), 6.16 (d, J = 2.1 Hz, 1H), 2.44 - 2.35 (m, 2H), 1.56 - 1.42 (m, 2H), 0.84 (t, J = 7.3 Hz, 3H). ¹³C-NMR (101 MHz, DMSO-d₆) δ 182.20, 164.66, 162.78, 161.93, 157.77, 148.25, 145.67, 123.83, 120.86, 118.66, 116.21, 116.02, 103.53, 99.01, 93.77, 27.07, 22.25, 14.53.

### EXAMPLE 3

### Synthesis of 2-(3,4-dihydroxyphenyl)-5,7-dihydroxy-3-propyl-4H-chromen-4-one (compound 5c, Scheme 1).

### Step 1. Synthesis of (E)-2-(3,4-dimethoxybenzylidene)-1-(2-hydroxy-4,6-dimethoxyphenyl)hexane-1-one (intermediate 3c, Scheme 1)

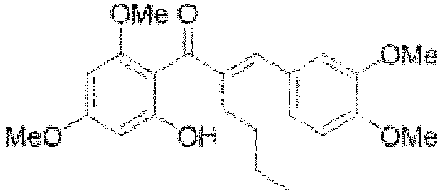

The title compound was synthesized following the general procedure 1 described above using 1-(2-hydroxy-4,6-dimethoxyphenyl)hexane-1-one **1c** (150 mg, 0.6 mmol), 3,4-dimethoxybenzaldehyde **2** (109 mg, 0.66 mmol) and potassium hydroxide (707 mg, 12.6 mmol) in MeOH (7 mL). Purification by silica (elution by gradient from 100 to 70/30 cyclohexane/EtOAc) gave the pure intermediate **3c** (81 mg, 35% yield).

**Characterization:** UPLC/MS Rt: 2.50 min (gradient 1), MS (ESI) m/z: 401.0 [M+H]⁺.[M+H]⁺ Calculated for C₂₃H₂₈O₆: 401.5. ¹H-NMR (400 MHz, DMSO-d₆) δ 9.70 (s, 1H), 7.06 - 6.97 (m, 2H), 6.94 (d, J = 2.0 Hz, 1H), 6.92 (d, J = 2.3 Hz, 1H), 6.12 (d, J = 2.1 Hz, 1H), 6.07 (d, J = 2.1 Hz, 1H), 3.78 (s, 3H), 3.76 (s, 3H), 3.74 (s, 3H), 3.65 (s, 3H), 2.62 - 2.53 (m, 2H), 1.51 - 1.42 (m, 2H), 1.42 - 1.31 (m, 2H), 0.90 (t, J = 7.2 Hz, 3H).

### Step 1. Synthesis of 3-butyl-2-(3,4-dimethoxyphenyl)-5,7-dimethoxy-4H-chromen-4-one (Compound 4c, Scheme 1).

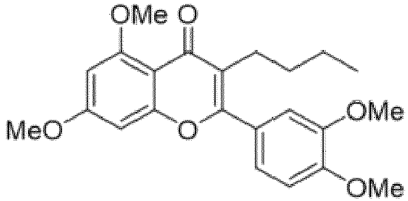

The title compound was synthesized following the general procedure 2 described above using intermediate **3c** (81 mg, 0.2 mmol) and I₂ (4 mg, 0.01 mmol) in DMSO (1 mL). Purification by silica (elution by gradient from 15/85 to 50/50 cyclohexane/EtOAc) gave the pure intermediate **4c** (48 mg, 61% yield).

**Characterization:** UPLC/MS Rt: 2.38 min (gradient 1), MS (ESI) m/z: 399.0 [M+H]⁺. [M+H]⁺ Calculated for C₂₃H₂₇O₆: 399.5. ¹H-NMR (400 MHz, DMSO-d₆) δ 7.18 - 7.15 (m, 2H), 7.11 (d, J = 8.8 Hz, 1H), 6.64 (d, *J* = 2.3 Hz, 1H), 6.48 (d, J = 2.3 Hz, 1H), 3.86 (s, 3H), 3.83 (s, 3H), 3.82 (s, 3H), 3.81 (s, 3H), 2.42 - 2.30 (m, 2H), 1.42 (p, J = 7.3 Hz, 2H), 1.23 (sextet, *J* = 7.3 Hz, 2H), 0.81 (t, *J* = 7.3 Hz, 3H) .

### Synthesis of 2-(3,4-dihydroxyphenyl)-5,7-dihydroxy-3-propyl-4H-chromen-4-one (compound 5c, Scheme 1).

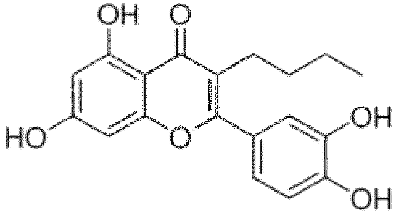

The title compound was synthesized following the general procedure 3 Method A described above using intermediate **4c** (48 mg, 0.12 mmol) and pyridinium chloride (139 mg, 1.2 mmol). Purification by silica (elution by gradient from 100 to 94:6 DCM/MeOH) gave the pure compound **5c** (10 mg, 30% yield).

**Characterization:** UPLC/MS Rt: 2.07 min (gradient 1), MS (ESI) m/z: [M+H]⁺. [M+H]⁺ Calculated for C₁₉H₁₉O₆: 343.4. ¹H-NMR (400 MHz, DMSO-d₆) δ 13.05 (s, 1H), 7.01 (d, J = 2.0 Hz, 1H), 6.92 (dd, J = 8.2, 2.0 Hz, 1H), 6.87 (d, J = 8.2, Hz, 1H), 6.25 (d, J = 2.0 Hz, 1H), 6.14(d, J = 2.0 Hz, 1H), 2.44 - 2.40 (m, 2H), 1.49 - 1.42 (m, 2H), 1.25 (m, 2H), 0.82 (t, J = 7.2 Hz, 3H).

### EXAMPLE 4

### Synthesis of 3-butoxy-2-(3,4-dihydroxyphenyl)-5,7-dihydroxy-4H-chromen-4-one (compound 9a, Scheme 2).

### Step 1. Synthesis of 7-(benzyloxy)-2-(3,4-bis(benzyloxy)phenyl)-5-hydroxy-3-(((2S,3R,4S,5S,6R)-3,4,5-trihydroxy-6-((((2R,3R,4R,5R,6S)-3,4,5-trihydroxy-6-methyltetrahydro-2H-pyran-2-yl)oxy)methyl)tetrahydro-2H-pyran-2-yl)oxy)-4H-chromen-4-one (compound 6, Scheme 2).

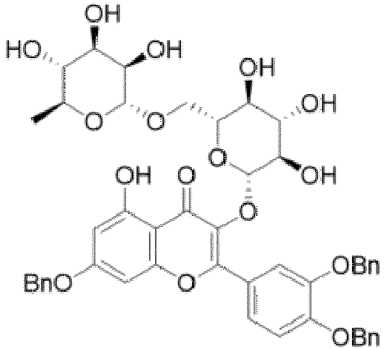

To a solution of rutin (2000 mg, 2.96 mmol) in DMF (20 mL) K₂CO₃ (1716 mg, 12.44 mmol) and benzoyl bromide (2.8 mL, 23.68 mmol) were added. The reaction mixture was stirred overnight at room temperature. Then it was diluted with EtOAc (60 mL). The organic phase was divided, washed with water (2x50 mL), dried over magnesium sulfate, filtered and concentrated under vacuum giving the crude product **6,** which was used without further purification for the next step (2000 mg).

**Characterization:** UPLC/MS Rt: 2.44 min (gradient 1), MS (ESI) m/z: 881.3 [M+H]⁺. [M+H]⁺ Calculated for C₄₈H₄₉O₁₆: 883.3.

### Step 2. Synthesis of 7-(benzyloxy)-2-(3,4-bis(benzyloxy)phenyl)-3,5-dihydroxy-4H-chromen-4-one (compound 7, Scheme 2).

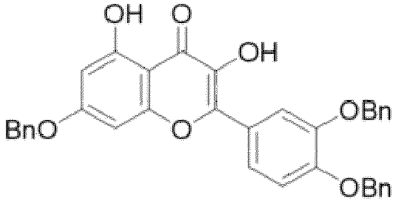

Intermediate **6** (2000 mg, 2.27 mmol) was dissolved in ethanol (14 mL) to which 37% HCl (2 mL) was added. The reaction mixture was refluxed for 120 minutes. After complete conversion of the starting material, the reaction mixture was cooled to room temperature and filtered. The precipitate was washed with water (5 mL) and cold MeOH (5 mL), giving the pure product **7** (1394 mg, yield: 82% in the two steps).

**Characterization:** UPLC/MS Rt: 2.55 min (gradient 2), MS (ESI) m/z: 573.2 [M+H]⁺. [M+H]⁺ Calculated for C₃₆H₂₉O₇: 573.2. ¹H-NMR (400 MHz, DMSO-d₆) δ 11.70 (s, 1H), 7.85 (d, J = 2.1 Hz, 1H), 7.77 (dd, J = 8.5, 2.0 Hz, 1H), 7.54 - 7.29 (m, 15H), 7.04 (d, J = 8.7 Hz, 1H), 6.52 (d, J = 2.2 Hz, 1H), 6.45 (d, J = 2.2 Hz, 1H), 5.25 (s, 4H), 5.15 (s, 2H).

### Step 3. Synthesis of 7-(benzyloxy)-2-(3,4-bis(benzyloxy)phenyl)-3-butoxy-5-hydroxy-4H-chromen-4-one (compound 8a, Scheme 2)

Compound **8a** was prepared following the general procedure 4 using: K₂CO₃ (207 mg, 3 mmol) and 4-bromobutane (0.07 mL, 0.7 mmol) were added to a solution of intermediate **7** (300 mg, 0.50 mmol) in DMF (8 mL). The crude product of compound **8a** was used as such without further purification.

**Characterization:** UPLC/MS Rt: 2.07 min (gradient 2), no ionization was detected.

### Step 4. Synthesis of 3-butoxy-2-(3,4-dihydroxyphenyl)-5,7-dihydroxy-4H-chromen-4-one (compound 9a, Scheme 2).

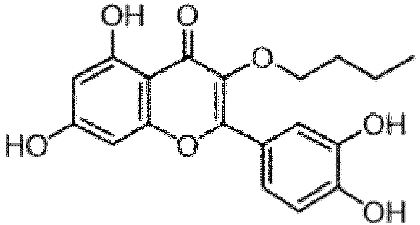

Compound **9a** was prepared following the general procedure 5 using: the crude product of compound **8a** (0.60 mmol), Pd/C (50 mg), Et₃SiH (2.0 mL, 10 mmol) in a 1:1 mixture of MeOH/DCM (15 mL). Purification by silica (elution by gradient from 100 to 50/50 Cyclohexane/EtOAc) gave the pure compound 9 (110 mg, 62% yield in two steps). **Characterization:** Rt: 1.40 min (gradient 1), MS (ESI) m/z: 359.1 [M+H]⁺.[M+H]⁺ Calculated for C₁₉H₁₉O₇: 359.1.

¹H-NMR (400 MHz, DMSO-d₆) δ 12.74 (s, 1H), 7.52 (d, J = 2.2 Hz, 1H), 7.44 (dd, J = 8.4, 2.2 Hz, 1H), 6.88 (d, J = 8.4 Hz, 1H), 6.44 - 6.36 (m, 1H), 6.20 - 6.15 (m, 1H), 3.92 (t, J = 6.6 Hz, 2H), 1.68 - 1.56 (m, 2H), 1.44 - 1.30 (m, 2H), 0.86 (t, J = 7.4 Hz, 3H).

### EXAMPLE 5

### Synthesis of 2-(3,4-dihydroxyphenyl)-5,7-dihydroxy-3-(2-hydroxyethoxy)-4H-chromen-4-one (compound 9b, Scheme 2).

Steps 1 and 2 of Example 4 are repeated and the following further steps are carried out:

### Step 3. Synthesis of 7-(benzyloxy)-2-(3,4-bis(benzyloxy)phenyl)-5-hydroxy-3-(2-hydroxyethoxy)-4H-chromen-4-one (compound 8b).

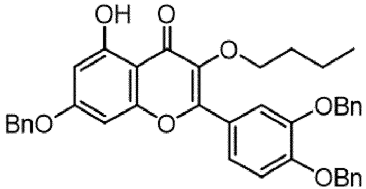

The compound **8b** was prepared following the general procedure 4 using: K₂CO₃ (124 mg, 0.9 mmol) and 2-bromoethanol (0.03 mL, 0.45 mmol) and intermediate 7 (200 mg, 0.30 mmol) in DMF (5 mL). The crude product of compound **8b** was used as such without further purification.

**Characterization:** UPLC/MS Rt: 0.58 min (gradient 2), MS (ESI) m/z: 615.4 [M - H]⁻. [M - H]⁻Calculated for C₃₈H₃₁O₈: 615.7.

### Step 4. Synthesis of 2-(3,4-dihydroxyphenyl)-5,7-dihydroxy-3-(2-hydroxyethoxy)-4H-chromen-4-one (compound 9b, Scheme 2)

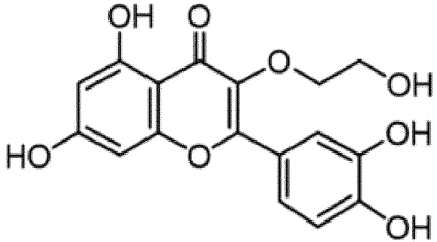

Compound **9b** was prepared following the general procedure 5 using: the crude product of compound **8b** (0.30 mmol), Pd/C (25 mg), Et₃SiH (1.2 mL, 6 mmol) in a 1:1 mixture of MeOH/DCM (8 mL). Purification by silica (elution by gradient from 100 to 30/70 Cyclohexane/EtOAc) gave the pure compound **9b** (33 mg, 15% yield in two steps). **Characterization:** Rt: 1.40 min (gradient 1), MS (ESI) m/z: 345.1 [M - H]-. [M - H]- Calculated for C₁₇H₁₃O₈: 345.3.

¹H-NMR (400 MHz, DMSO-d₆) δ 12.68 (s, 1H), 7.57 (dd, J = 9.5, 2.2 Hz, 1H), 7.56 (s, 1H), 6.86 (d, J = 8.2 Hz, 1H), 6.36 (d, J = 2.1 Hz, 1H), 6.15 (d, J = 2.1 Hz, 1H), 3.99 (t, J = 5.3 Hz, 2H), 3.65 (t, J = 5.3 Hz, 2H).

### EXAMPLE 6

### Synthesis of 2-(3,4-dihydroxyphenyl)-5,7-dihydroxy-3-(3-hydroxypropoxy)-4H-chromen-4-one (compound 9c, Scheme 2)

Steps 1 and 2 of Example 4 are repeated and the following further steps are carried out:

### Step 3. Synthesis of 7-(benzyloxy)-2-(3,4-bis(benzyloxy)phenyl)-5-hydroxy-3-(3-hydroxypropoxy)-4H-chromen-4-one (compound 8c, Scheme 2) .

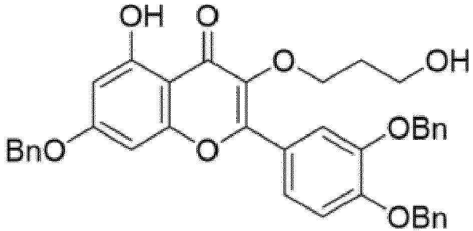

Compound **8c** was prepared following the general procedure 4 using: K₂CO₃ (248 mg, 0.18 mmol), 3-bromo-1-propanol (0.14 mL, 0.9 mmol), and intermediate **7** (350 mg, 0.60 mmol) in DMF (10 mL). The crude product of intermediate **8c** was used as such without further purification.

**Characterization:** UPLC/MS Rt: 2.40 min (gradient 2), MS (ESI) m/z: 631.2 [M+H]⁺.[M+H]⁺ Calculated: 631.2 for C₄₁H₃₇O₈.

### Step 4. Synthesis of 2-(3,4-dihydroxyphenyl)-5,7-dihydroxy-3-(3-hydroxypropoxy)-4H-chromen-4-one (compound 9c, Scheme 2).

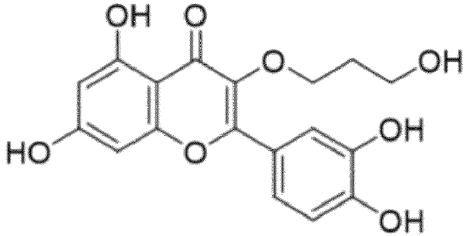

Compound **9c** was prepared following the general procedure 5 using: compound **8c** (365 mg, 0.58 mmol), Pd/C (72 mg), Et₃SiH (2.2 mL, 10.5 mmol) in a 1:1 mixture of MeOH/DCM (14 mL). Purification by silica (elution by gradient from 100 to 40/60 Cyclohexane/EtOAc) gave the pure compound **9c** (33 mg, 15% yield in two steps). **Characterization:** Rt: 1.40 min (gradient 1), MS (ESI) m/z: 361.1 [M+H]⁺.[M+H]⁺ Calculated for C₁₉H₁₉O₇: 361.1.

¹H-NMR (400 MHz, DMSO-d₆) δ 12.72 (s, 1H), 7.53 (d, J = 2.2 Hz, 1H), 7.46 (dd, J = 8.4, 2.2 Hz, 1H), 6.89 (d, J = 8.4 Hz, 1H), 6.39 (d, J = 2.1 Hz, 1H), 6.18 (d, J = 2.1 Hz, 1H), 4.01 (t, J = 6.7 Hz, 2H), 3.49 (t, J = 6.4 Hz, 2H), 1.81 (p, J = 6.5 Hz, 2H). ¹³C-NMR (101 MHz, DMSO-d₆) δ 178.04, 164.12, 161.29, 156.37, 155.95, 148.62, 145.18, 136.75, 120.96, 120.78, 115.64, 115.51, 104.17, 98.54, 93.57, 69.72, 57.71, 32.91.

### EXAMPLE 7

### Synthesis of 2-(3,4-dihydroxybenzyl)-5-hydroxy-4H-chromen-4-one (compound 13a, Scheme 3)

### Step 1: Synthesis of 4-(3,4-dimethoxyphenyl)-1-(2-hydroxy-6-methoxyphenyl)butane-1,3-dione (compound 11a, Scheme 3).

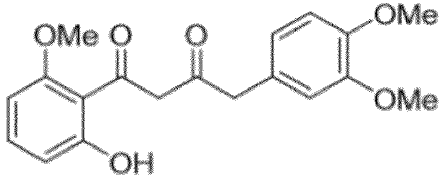

Compound **11a** was prepared following the general procedure 7 method A using: 6'-hydroxy-2'-methoxyacetophenone **10a** (200 mg, 1.2 mmol), ethyl 2-(3,4-dimethoxyphenyl)acetate (538 mg, 2.4 mmol), a 60% NaH dispersion in mineral oil (192 mg, 4.8 mmol) in anhydrous THF (6 mL). The crude product was used as such without further purification. **Characterization:** UPLC/MS Rt: 1.85 min (gradient 1); MS (ESI) m/z: 345.0 [M+H]⁺, [M+H]⁺ Calculated for C₁₉H₂₁O₆: 345.4.

### Step 2: Synthesis of 2-(3,4-dimethoxybenzyl)-5-methoxy-4H-chromen-4-one (compound 12a, Scheme 3).

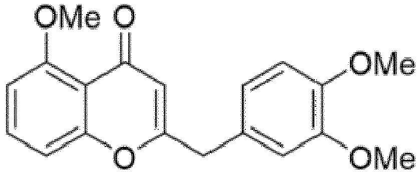

Compound **11** was prepared following the general procedure 7 using: crude product **11a** (412 mg), 37% HCl (1 mL) in MeOH (7 mL). The reaction mixture was stirred at room temperature for 20 hours. Purification by silica (elution by gradient from 100:0 to 50:50 dichloromethane/EtOAc) gave the pure title compound **12a** as a white powder (145 mg, 37% yield in 2 steps). **Characterization:** UPLC/MS Rt: 1.80 min (gradient 1), MS (ESI) m/z 327.1, [M+H]⁺. [M+H]⁺ calculated for C₁₉H₁₉O5: 327.3. ¹H NMR (400 MHz, CDCl₃) δ 7.50 (t, J = 8.41 Hz, 1H), 6.96 (d, J = 8.35 Hz, 1H), 6.83 (bs, 2H), 6.79-6.77 (m, 2H), 6.06 (s, 1H), 3.95 (s, 3H), 3.97 (s, 3H), 3.86 (s, 3H), 3.48 (s, 2H).

### Step 3: Synthesis of 2-(3,4-dihydroxybenzyl)-5-hydroxy-4H-chromen-4-one (compound 13a, Scheme 3).

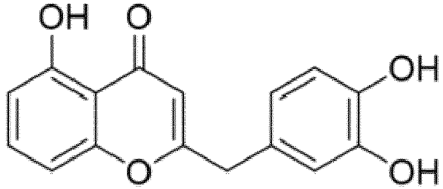

Compound **13a** was prepared according to general procedure C method B using: intermediate **12a** (150 mg, 0.46 mmol), BBrs (1 M in DCM) (2.1 mL, 2.06 mmol) in anhydrous CH₂Cl₂ (6 mL). The crude product was purified by flash chromatography on silica gel (elution by gradient from 100:0 to 90:10 dichloromethane/EtOAc) to give product **13a** (120 mg, 92% yield). **Characterization:** UPLC/MS Rt: 1.77 min (gradient 1), MS (ESI) m/z 285.0, [M+H]⁺. [M+H]⁺ calculated for C₁₆H₁₃O₅: 284.3. ¹H NMR (400 MHz, DMSO-d₆) δ 12.60 (s, 1H), 8.81 (s, 1H), 7.61 (t, *J* = 8.4 Hz, 1H), 6.99 (dd, *J*= 8.5, 0.9 Hz, 1H), 6.78 (dd, *J* = 8.3, 0.9 Hz, 1H), 6.72 (d, *J* = 2.2 Hz, 1H), 6.70 (d, *J =* 8.0 Hz, 1H), 6.60 (dd, *J*= 8.0, 2.1 Hz, 1H), 6.24 (s, 1H), 3.85 (s, 2H) .

### Example 8

### Synthesis of 2-(1-(3,4-dihydroxyphenyl)ethyl)-5-hydroxy-4H-chromen-4-one (compound 13b, Scheme 3)

### Step 1: Synthesis of methyl-2-(3,4-dimethoxyphenyl)propanoate (compound 10b, Scheme 3)

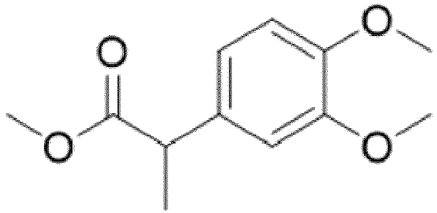

Compound **10b** was prepared according to general procedure 6 using: **10a** (400 mg, 1.9 mmol), LiHMDS (540 µL, 2.85 mmol), iodomethane (260 µL, 3.8 mmol), anhydrous THF (1.0 mL). The crude material was purified by flash chromatography on silica gel (elution by gradient from 100:0 to 75:25 cyclohexane/AcOEt) to obtain product **10b** (306 mg, 72% yield). **Characterization:** UPLC/MS Rt: 1.74 min (gradient 1), MS (ESI) m/z 225.1, [M+H]⁺. [M+H]⁺ calculated for C₁₂H₁₇O₄: 225.1. ¹H NMR (400 MHz, DMSO-d₆) δ 6.89 (d, J = 8.2 Hz, 1H), 6.85 (d, J = 2.1 Hz, 1H), 6.77 (dd, J = 8.2, 2.1 Hz, 1H), 3.76 - 3.68 (m, 7H), 3.57 (s, 3H), 1.36 (d, J = 7.1 Hz, 3H).

### Step 2: Synthesis of 4-(3,4-dimethoxyphenyl)-1-(2-hydroxy-6-methoxyphenyl)pentane-1,3-dione (compound 11b, Scheme 3)

Compound **11b** was prepared following the general procedure 7 method B using: 1-(2-hydroxy-6-methoxyphenyl)ethane-1-one (56 mg, 0.34 mmol), **10b** (77 mg, 0.348 mmol), LDA (0.51 µL, 1.02 mmol) in anhydrous THF (2.8 mL). The crude product was filtered on silica (elution by gradient 90:10 to 70:30 cyclohexane/AcOEt) to remove much more polar and/or apolar impurities and obtain a cleaner crude material for use in the next step. **Characterization:** UPLC/MS Rt: 2.02 min (gradient 1); MS (ESI) m/z: 359.1 [M+H]⁺, [M+H]⁺ Calculated for C₂₀H₂₃O₆: 359.1.

### Step 3: Synthesis of 2-(1-(3,4-dimethoxyphenyl)ethyl)-5-methoxy-4H-chromen-4-one (compound 12b, Scheme 3)

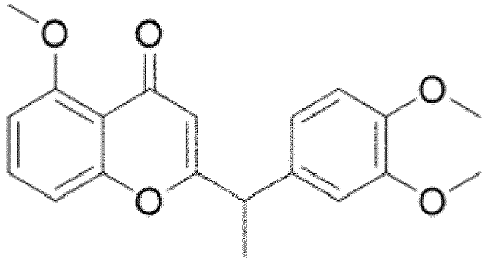

Compound **12b** was prepared according to general procedure 8 method B using: crude material from the previous step (34 mg, 0.095 mmol), CH₃COOH (0.45 mL), H₂SO₄ (2 drops). The new crude material was purified by flash chromatography on silica gel (elution by gradient from 60:40 to 0:100 cyclohexane/AcOEt) to obtain product **12b** (28 mg, with overall yield of the 2 steps of 24%). **Characterization:** ¹H NMR (400 MHz, DMSO-d₆) δ 7.61 (t, J = 8.4 Hz, 1H), 7.03 (d, J = 8.4 Hz, 1H), 6.99 - 6.85 (m, 4H), 6.06 (s, 1H), 4.06 - 3.97 (m, 1H), 3.82 (s, 3H), 3.74 (s, 3H), 3.72 (s, 3H), 1.54 (d, J = 7.2 Hz, 3H).

### Step 4: Synthesis of 2-(1-(3,4-dihydroxyphenyl)ethyl)-5-hydroxy-4H-chromen-4-one (compound 13b, Scheme 4)

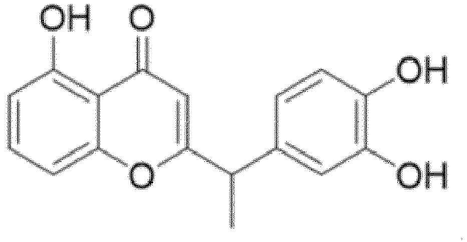

Compound **13b** was prepared according to general procedure 3 method B using: intermediate **12b** (28 mg, 0.082 mmol), BBrs (1 M in DCM) (0.37 mL, 0.37 mmol) in anhydrous CH₂Cl₂ (3.1 mL). The crude product was purified by flash chromatography on silica gel (elution by gradient from 100:0 to 98:2 dichloromethane/MeOH) to give product **13b** (19 mg, 78% yield). **Characterization:** UPLC/MS Rt: 1.93 min (gradient 1), MS (ESI) m/z 297.2, [M-H]⁻; 299.0, [M-H]⁺. [M-H]⁺ calculated for C₁₇H₁₄O₅: 299.1.0. ¹H NMR (400 MHz, DMSO-d₆) δ 7.61 (t, J = 8.4 Hz, 1H), 6.99 (dd, J = 8.5, 0.9 Hz, 1H), 6.78 (d, J = 7.9 Hz, 1H), 6.74 (d, J = 2.2 Hz, 1H), 6.69 (d, J = 8.1 Hz, 1H), 6.63 (dd, J = 8.1, 2.1 Hz, 1H), 6.33 (s, 1H), 4.01 (q, J = 7.2 Hz, 1H), 1.53 (d, J = 7.1 Hz, 3H) .

### Example 9

### Synthesis of 2-(1-(3,4-dihydroxyphenyl)pentyl)-5-hydroxy-4H-chromen-4-one (compound 13c, Scheme 4)

### Step 1: Synthesis of methyl-2-(3,4-dimethoxyphenyl)hexanoate (compound 10c, Scheme 4)

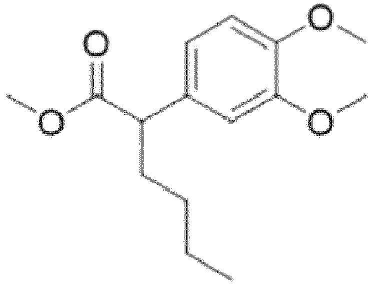

Compound **10c** was prepared according to general procedure 6 using: **10a** (300 mg, 1.43 mmol), LiHMDS (400 µL, 2.14 mmol), 1-iodobutane (230 µL, 2.85 mmol), anhydrous THF (0.71 mL). The crude material was purified by flash chromatography on silica gel (elution by gradient from 100:0 to 85:15 cyclohexane/AcOEt) to obtain product **10c** (228 mg, 60% yield). **Characterization:** UPLC/MS Rt: 2.27 min (gradient 1), MS (ESI) m/z 267.1, [M+H]+. [M+H]+ calculated for C₁₅H₂₃O₄: 267.2. 1H NMR (400 MHz, DMSO-d6) δ 6.89 (d, J = 8.2 Hz, 1H), 6.85 (d, J = 2.0 Hz, 1H), 6.78 (dd, J = 8.2, 2.0 Hz, 1H), 3.73 (s, 3H), 3.72 (s, 3H), 3.57 (s, 3H), 3.52 (t, J = 7.7 Hz, 1H), 1.99 - 1.85 (m, 1H), 1.71 - 1.58 (m, 1H), 1.33 - 1.06 (m, 4H), 0.82 (t, J = 7.2 Hz, 3H).

### Step 2: Synthesis of 2-(3,4-dimethoxyphenyl)hexanoic acid (compound 14c, Scheme 4)

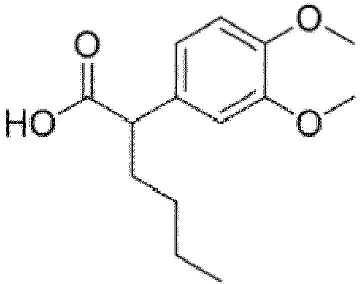

Compound **14c** was prepared according to general procedure 9 using: **10c** (330 mg, 1.03 mmol), LiOH (49 mg, 2.06 mmol), THF (9.3 mL), H₂O (1.0 mL). The crude material was purified by flash chromatography on silica gel (elution by gradient from 70:30 to 30:70 cyclohexane/DCM and then from 50:50 to 0:100 DCM/AcOEt) to obtain product **14c** (234 mg, 90% yield). **Characterization:** UPLC/MS Rt: 1.85 min (gradient 1), [MS (ESI) m/z 251.2, [M-H]^{-,}253.1, [M+H]⁺. [M+H]⁺ calculated for C₁₄H₂₁O₄: 253.1. ¹H NMR (400 MHz, DMSO-d₆) δ 6.88 (d, J = 8.3 Hz, 1H), 6.86 (d, J = 2.1 Hz, 1H), 6.79 (dd, J = 8.2, 2.1 Hz, 1H), 3.72 (s, 3H), 3.72 (s, 3H), 1.97 - 1.83 (m, 1H), 1.66 - 1.53 (m, 1H), 1.35 - 1.06 (m, 4H), 0.83 (t, J = 7.2 Hz, 3H).

### Step 3: Synthesis of -(3,4-dimethoxyphenyl)-1-(2-hydroxy-6-methoxyphenyl)octane-1,3-dione (compound 11c, Scheme 4)

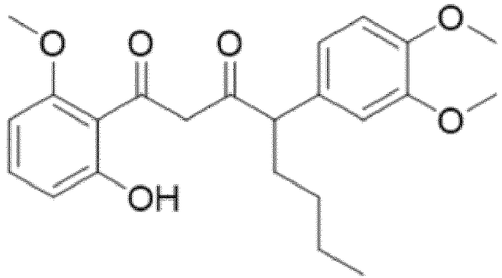

Compound **11c** was prepared following the general procedure 7 method C: using 1-(2-hydroxy-6-methoxyphenyl)ethane-1-one (60 mg, 0.36 mmol), LDA (0.54 mL, 1.08 mmol) in anhydrous THF (3 mL) to obtain the enolate to which the freshly prepared acyl chloride using **14c** (100 mg, 0.396 mmol), SOCl₂ (290 µL, 3.96 mmol) in anhydrous DCM (1.5 mL) was added. The crude product was filtered on silica (elution by gradient from 100:0 to 50:50 cyclohexane/AcOEt) to remove much more polar and/or apolar impurities and obtain a cleaner crude material to be used in the next step. **Characterization:** UPLC/MS Rt: 1.67 min (gradient 2); MS (ESI) m/z: 399.2 [M-H]⁻; 401.1 [M+H]⁺, [M+H]⁺ Calculated for C₂₃H₂₉O₆: 401.2.

### Step 4: Synthesis of 2-(1-(3,4-dimethoxyphenyl)pentyl)-5-methoxy-4H-chromen-4-one (compound 12c, Scheme 4)

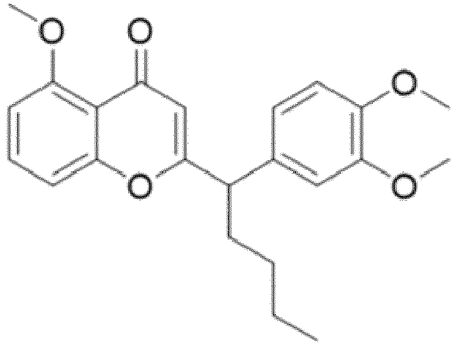

Compound **12c** was prepared according to general procedure 8 method B using: intermediate **11c** (65 mg, 0.15 mmol), CH₃COOH (0.5 mL), H₂SO₄ (2 drops). The crude material was purified by flash chromatography on silica gel (elution by gradient 90:10 to 0:100 cyclohexane/AcOEt) to obtain product **12c** (40 mg, 29% 2-step overall yield). **Characterization:** UPLC/MS Rt: 1.21 min (gradient 2), MS (ESI) m/z 383.1, [M+H]⁺. [M+H]⁺ calculated for C₂₃H₂₇O₅: 383.2. ¹H NMR (400 MHz, DMSO-d₆) δ 7.61 (t, J = 8.4 Hz, 1H), 7.08 (dd, J = 8.5, 0.9 Hz, 1H), 6.99 (d, J = 1.6 Hz, 1H), 6.95 - 6.88 (m, 3H), 6.13 (s, 1H), 3.84 - 3.77 (m, 4H), 3.75 (s, 3H), 3.72 (s, 3H), 2.13 - 2.01 (m, 1H), 1.96 - 1.84 (m, 1H), 1.38 - 1.26 (m, 2H), 1.26 - 1.15 (m, 2H), 0.84 (t, J = 7.2 Hz, 3H).

### Step 5: Synthesis of 2-(1-(3,4-dihydroxyphenyl)pentyl)-5-hydroxy-4H-chromen-4-one (compound 13c, Scheme 4)

Compound **13c** was prepared according to general procedure 3 method B using: intermediate **12c** (35 mg, 0.092 mmol), BBrs (1 M in DCM) (0.28 mL, 0.28 mmol) in anhydrous CH₂Cl₂ (1.9 mL). The crude product was purified by flash chromatography on silica gel (elution by gradient from 100:0 to 98:2 dichloromethane/MeOH) to give product **13c** (29 mg, 92% yield). **Characterization:** UPLC/MS Rt: 1.21 min (gradient 2), MS (ESI) m/z 339.2, [M-H]⁻; 341.1, [M+H]⁺. [M-H]⁺ calculated for C₂₀H₂₁O₅: 341.1. ¹H NMR (400 MHz, DMSO-d₆) δ 12.59 (s, 1H), 8.88 (s, 2H), 7.61 (t, J = 8.3 Hz, 1H), 7.01 (dd, J = 8.5, 0.9 Hz, 1H), 6.79 - 6.74 (m, 2H), 6.71 - 6.64 (m, 2H), 6.39 (s, 1H), 3.80 (t, J = 7.8 Hz, 1H), 2.11 - 1.97 (m, 1H), 1.93 - 1.80 (m, 1H), 1.37 - 1.26 (m, 2H), 1.25 - 1.14 (m, 2H), 0.83 (t, J = 7.2 Hz, 3H).

### Example 10

### Synthesis of 2-(1-(3,4-dihydroxyphenyl)-5,5,5-trifluoropentyl)-5-hydroxy-4H-chromen-4-one (compound 13d, Scheme 4)

### Step 1: Synthesis of methyl-2-(3,4-dimethoxyphenyl)-6,6,6-trifluorohexanoate (compound 10d, Scheme 4)

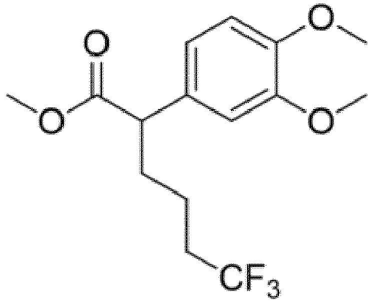

Compound **10d** was prepared according to general procedure 6 using: **10a** (400 mg, 1.9 mmol), LDA (1.4 mL, 2.85 mmol), 1,1,1-trifluoro-4-iodobutane (270 µL, 2.09 mmol), anhydrous THF (1.0 mL). The crude material was purified by flash chromatography on silica gel (elution by gradient from 100:0 to 80:20 cyclohexane/AcOEt) to obtain product **10d** (339 mg, 55% yield). **Characterization:** UPLC/MS Rt: 2.35 min (gradient 2), [MS (ESI) m/z 321.1, [M+H]⁺. [M+H]⁺ calculated for C₁₅H₂₀F₃O₄: 321.1. ¹H NMR (400 MHz, DMSO-d₆) δ 6.90 (d, J = 8.3 Hz, 1H), 6.86 (d, J = 2.0 Hz, 1H), 6.79 (dd, J = 8.2, 2.0 Hz, 1H), 3.73 (s, 3H), 3.72 (s, 3H), 3.64 - 3.56 (m, 4H), 2.32 - 2.16 (m, 2H), 2.05 - 1.93 (m, 1H), 1.81 - 1.70 (m, 1H), 1.46 - 1.27 (m, 2H).

### Step 2 : Synthesis of 2-(3,4-dimethoxyphenyl)-6,6,6-trifluorohexanoic acid (compound 14d, Scheme 4)

Compound **14d** was prepared according to general procedure 9 using: **10d** (330 mg, 1.03 mmol), LiOH (49 mg, 2.06 mmol), THF (9.3 mL), H₂O (1.0 mL) . The crude material was purified by flash chromatography on silica gel (elution by gradient from 70:30 to 30:70 cyclohexane/DCM and then from 50:50 to 0:100 DCM/AcOEt) to obtain product **14d** (278 mg, 88% yield). **Characterization:** ¹H NMR (400 MHz, DMSO-d₆) δ 6.90 (d, J = 8.3 Hz, 1H), 6.87 (d, J = 2.0 Hz, 1H), 6.80 (dd, J = 8.2, 2.1 Hz, 1H), 3.73 (s, 3H), 3.72 (s, 3H), 3.47 (t, J = 7.7 Hz, 1H), 2.31 - 2.16 (m, 2H), 2.05 - 1.90 (m, 1H), 1.76 - 1.65 (m, 1H), 1.49 - 1.30 (m, 2H).

### Step 3: Synthesis of 4-(3,4-dimethoxyphenyl)-8,8,8-trifluoro-1-(2-hydroxy-6-methoxyphenyl)octane-1,3-dione (compound 11d, Scheme 4)

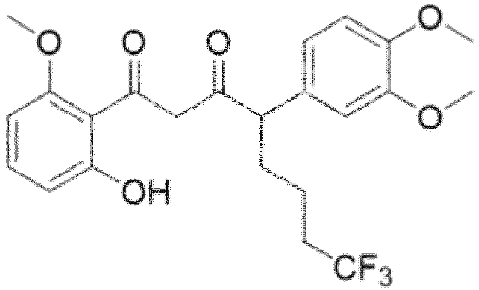

Compound **11d** was prepared following the general procedure 7 method C using: 1-(2-hydroxy-6-methoxyphenyl)ethane-1-one (50 mg, 0.30 mmol), LDA (0.45 mL, 0.90 mmol) in anhydrous THF (4.9 mL) to obtain the enolate to which the freshly prepared acyl chloride using **14d** (138 mg, 0.45 mmol), SOCl₂ (330 µL, 4.5 mmol) in anhydrous DCM (1.7 mL) was added. The crude product was filtered on silica (elution by gradient 100:0 to 80:50 cyclohexane/AcOEt) to remove much more polar and/or apolar impurities and obtain a cleaner crude material for use in the next step. **Characterization:** UPLC/MS Rt: 1.48 min (gradient 2); MS (ESI) m/z: 453.3 [M-H]⁻, 455.1 [M+H]⁺, [M+H]⁺ Calculated for C₂₃H₂₆F₃O₆: 455.2.

### Step 4: Synthesis of 2-(1-(3,4-dimethoxyphenyl)-5,5,5-trifluoropentyl)-5-methoxy-4H-chromen-4-one (compound 12d, Scheme 4)

Compound **12d** was prepared according to general procedure 8 method B using: intermediate **11d** (70 mg, 0.154 mmol), CH₃COOH (0.6 mL), H₂SO₄ (2 drops). The crude material was purified by flash chromatography on silica gel (elution by gradient 80:20 to 0:100 cyclohexane/AcOEt) to obtain product **12d** (32 mg, with overall yield of the 2 steps of 24%). **Characterization:** UPLC/MS Rt: 1.03 min (gradient 2), MS (ESI) m/z 435.2 [M-H]⁻; 437.1, [M+H]⁺. [M+H]⁺ calculated for C₂₃H₂₄F₃O₅: 437.2. ¹H NMR (400 MHz, DMSO-d₆) δ 7.62 (t, J = 8.4 Hz, 1H), 7.06 (dd, J = 8.5, 0.9 Hz, 1H), 7.00 (s, 1H), 6.97 - 6.91 (m, 3H), 6.15 (s, 1H), 3.89 (t, J = 7.8 Hz, 1H), 3.82 (s, 3H), 3.75 (s, 3H), 3.72 (s, 3H), 2.40 - 2.23 (m, 2H), 2.20 - 2.08 (m, 1H), 2.07 - 1.94 (m, 1H) .

### Step 5: Synthesis of 2-(1-(3,4-dihydroxyphenyl)-5,5,5-trifluoropentyl)-5-hydroxy-4H-chromen-4-one (compound 13d, Scheme 4)

Compound **13d** was prepared according to general procedure 3 method B using: intermediate **12d** (35 mg, 0.080 mmol), BBrs (1 M in DCM) (0.25 mL, 0.25 mmol) in anhydrous CH₂Cl₂ (1.4 mL). The crude product was purified by flash chromatography on silica gel (elution by gradient from 100:0 to 99:1 dichloromethane/MeOH) to give product **13d** (28 mg, 88% yield). **Characterization:** UPLC/MS Rt: 1.03 min (gradient 2), MS (ESI) m/z 393.2, [M-H]⁻; 395.0, [M+H]⁺ [M+H]⁺ calculated for C₂₀H₁₈F₃O₅: 395.1. ¹H NMR (400 MHz, DMSO-d₆) δ 12.56 (s, 1H), 8.92 (s, 2H), 7.62 (t, J = 8.3 Hz, 1H), 7.00 (dd, J = 8.4, 0.9 Hz, 1H), 6.80 - 6.75 (m, 2H), 6.73 - 6.64 (m, 2H), 6.41 (s, 1H), 3.89 (t, J = 7.8 Hz, 1H), 2.40 - 2.22 (m, 2H), 2.19 - 2.05 (m, 1H), 2.03 - 1.89 (m, 1H), 1.52 - 1.37 (m, 2H).

### EXAMPLE 11

### Synthesis of (E)-2-(3,4-dihydroxystyryl)-5-hydroxy-4H-chromen-4-one (compound 18, Scheme 5)

### Step 1: Synthesis of 1-(2-hydroxy-6-methoxyphenyl)butane-1,3-dione (compound 15).

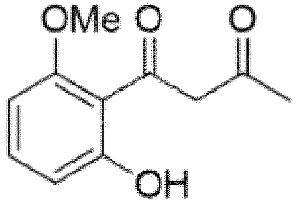

Compound **15** was prepared following the general procedure 7 method A using: 1-(2-hydroxy-6-methoxyphenyl)ethane-1-one (1 mL, 8.3 mmol), a 60% NaH dispersion in mineral oil (1328 mg, 33.2 mmol) in a 5:1 mixture of THF/EtOAc (24 mL). The crude product obtained was used as such, without further purification.

**Characterization:** UPLC/MS Rt: 1.42 min (gradient 1), MS (ESI) m/z 285.0, [M-H]⁻. [M-H]⁻ calculated for C₁₁H₁₁O₄: 207.2.

### Step 2: Synthesis of 5-methoxy-2-methyl-4H-chromen-4-one (compound 16, Scheme 5).

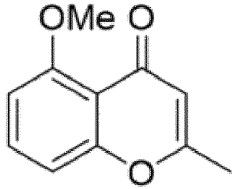

Compound **16** was prepared following the general procedure 8 method A using: the crude product of compound **15** (1480 g), 37% HCl (1 mL), in MeOH (20 mL). Purification by typical silica gel (elution by gradient 100:0 to 75:25 cyclohexane/EtOAc) gave the pure title compound **16** as a white powder (1.18 mg, 84% yield in 2 steps). **Characterization:** UPLC/MS Rt: 1.43 min (gradient 1), MS (ESI) m/z 190.9, [M+H]⁺. [M+H]⁺ calculated for C₁₁H₁₁O₃: 191.2. ¹H NMR (400 MHz, CDCl₃) δ 7.51 (t, *J* = 8.4 Hz, 1H), 6.98 (dd, *J* = 8.5, 1.0 Hz, 1H), 6.78 (dd, *J* = 8.4, 1.0 Hz, 1H), 6.10 (d, *J* = 0.9 Hz, 1H), 3.97 (s, 3H), 2.31 (d, *J* = 0.7 Hz, 3H).

### Step 3: Synthesis of (E)-2-(3,4-dimethoxystyryl)-5-methoxy-4H-chromen-4-one (Compound 17, Scheme 5).

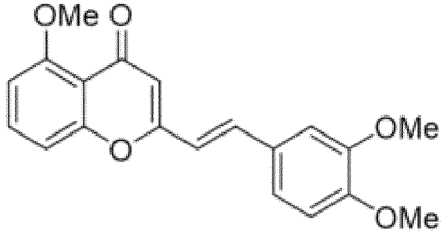

Sodium ethoxide (55 mg, 0.31 mmol) was added to a solution of compound **16** (100 mg, 0.62 mmol) in EtOH (2 mL), followed by addition of a solution of 3,4-dimethoxybenzaldehyde (110 mg, 0.62 mmol) in EtOH (1 mL). The reaction mixture was stirred at 50°C for 2 hours. After that, acq. HCl (2 M) was added to the reaction mixture to reach pH=4, the resulting precipitate was filtered and washed with water (0.5 mL) and dried under vacuum to give the pure title compound **17** (140 mg, 74% yield) . **Characterization:** UPLC/MS Rt: 1.97 min (gradient 1), MS (ESI) m/z 339.0, [M+H]⁺. [M+H]⁺ calculated for C₂₀H₁₉O₅: 338. 4. ¹H NMR (400 MHz, CDCl₃) δ 7.57 (t, *J* = 8.4 Hz, 1H), 7.51 (d, *J* = 16.0 Hz, 1H), 7.15 (dd, *J* = 8.4, 2.0 Hz, 1H), 7.10 (dd, J = 8.4, 1.0 Hz, 1H), 7.09 (d, J = 2.3 Hz, 1H), 6.90 (d, *J* = 8.3 Hz, 1H), 6.82 (dd, *J* = 8.4, 0.9 Hz, 1H), 6.61 (d, *J* = 16.0 Hz, 1H), 6.38 (s, 1H), 3.99 (s, 3H), 3.97 (s, 3H), 3.93 (s, 3H).

### Step 4. Synthesis of (E)-2-(3,4-dihydroxystyryl)-5-hydroxy-4H-chromen-4-one (Compound 18, Scheme 5).

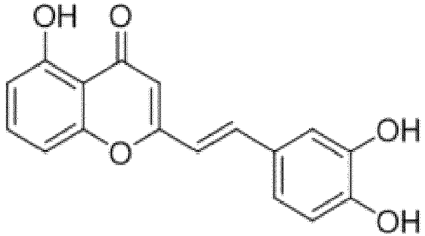

Compound **18** was prepared following the general procedure 3, method B using: intermediate **17** (89 mg, 0.26 mmol), BBrs (1 M in DCM) (1.2 mL, 1.17 mmol) in anhydrous DCM (5.2 mL). The crude product was purified by silica gel (elution by gradient from 100:0 to 80:20 DCM/EtOAc) to give product **18** (53 mg, 66% yield). **Characterization:** UPLC/MS Rt: 1.94 min (gradient 1), MS (ESI) m/z 297.0, [M+H]⁺. [M+H]⁺ calculated for C₁₇H₁₃O₅: 297.3. ¹H NMR (400 MHz, CDCl₃) δ 12.80 (s, 1H), 7.64 (t, *J* = 8.4 Hz, 1H), 7.59 (d, *J* = 16.0 Hz, 1H), 7.15 - 7.09 (m, 2H), 7.05 (dd, *J* = 8.2, 2.0 Hz, 1H), 6.88 (d, *J* = 16.0 Hz, 1H), 6.81 (d, *J* = 8.1 Hz, 1H), 6.78 (d, *J* = 8.1 Hz, 1H), 6.47 (s, 1H).

### EXAMPLE 12

### Synthesis of 2-(3,4-dihydroxyphenethyl)-5-hydroxy-4H-chromen-4-one (Compound 20, Scheme 5).

Steps 1 to 3 of Example 11 are conducted followed by the further steps below.

### Step 4: Synthesis of 2-(3,4-dimethoxyphenethyl)-5-methoxy-4H-chromen-4-one (compound 19).

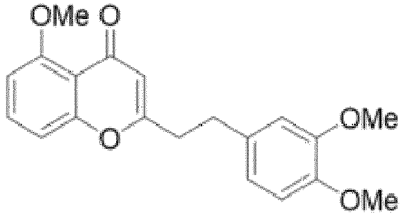

Ammonium formate (21 mg, 0.30 mmol) and Pd(OH)₂/C (0.015 g, 15% w/w) were added to a solution of intermediate **17** (100 mg, 0.29 mmol) in MeOH (3 mL). The reaction mixture was stirred at 80°C for 4 hours. Thereafter, the mixture was filtered over a pad of celite, increased with MeOH (10 mL), and concentrated under vacuum. Purification by silica (elution by gradient from 100:0 to 60:40 DCM/EtOAc) gave the pure title compound **19** (64mg, 63%). **Characterization:** UPLC/MS Rt: 1.89 min (gradient 1), MS (ESI) m/z 341.0, [M+H]⁺. [M+H]⁺ calculated for C₂₀H₂₁O₅: 341.4. ¹H NMR (400 MHz, CDCl₃) δ 7.52 (dd, *J* = 8.4, 8.4 Hz, 1H), 6.99 (dd, *J* = 8.4, 0.9 Hz, 1H), 6.81 - 6.76 (m, 2H), 6.75 - 6.69 (m, 2H), 6.08 (s, 1H), 3.97 (s, 3H), 3.85 (s, 3H), 3.82 (s, 3H), 2.97 (dd, *J* = 9.4, 7.3 Hz, 2H), 2.84 (dd, *J* = 9.3, 7.2 Hz, 2H).

### Step 5: Synthesis of 2-(3,4-dihydroxyphenethyl)-5-hydroxy-4H-chromen-4-one (compound 20, Scheme 5).

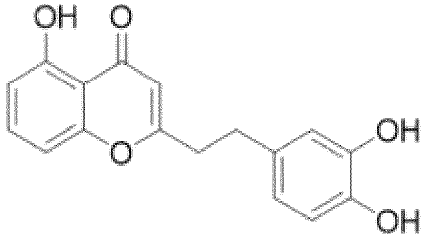

Compound **20** was prepared following the general procedure 3, method B using: intermediate **19** (64 mg, 0.19 mmol), BBrs (1 M in DCM) (0.86 mL, 0.68 mmol) in anhydrous DCM (3.8 mL). The crude product was purified by silica gel (elution by gradient from 100:0 to 85:15 DCM/EtOAc) to give product **20** (45 mg, 80% yield). **Characterization:** UPLC/MS Rt: 1.89 min (gradient 1), MS (ESI) m/z 299.0, [M+H]⁺. [M+H]⁺ calculated for C₁₇H₁₅O₅: 299.3. ¹H NMR (400 MHz, DMSO-d₆) δ 7.63 (dd, *J* = 8.4, 8.4 Hz, 1H), 7.04 (dd, *J* = 8.5, 0.9 Hz, 1H), 6.78 (dd, *J* = 8.2, 0.9 Hz, 1H), 6.62 (d, J = 5.1 Hz, 1H), 6.60 (bs, 1H), 6.47 (dd, *J* = 8.0, 2.1 Hz, 1H), 6.26 (s, 1H), 2.91 (ddd, *J* = 8.7, 6.8, 1.7 Hz, 2H), 2.84 (ddd, *J* = 8.4, 6.7 Hz, 1.8 Hz, 2H).

### EXAMPLE 13

### Synthesis of N-(3,4-dihydroxyphenyl)-5-hydroxy-4-oxo-4H-chromen-2-carboxamide (compound 24a, Scheme 5).

### Step 1. Synthesis of ethyl 5-methoxy-4-oxo-4H-chromen-2-carboxylate (compound 21, Scheme 6).

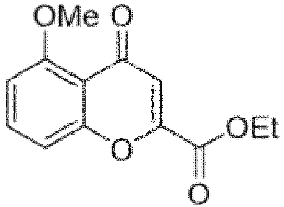

NaOEt (210 mg, 3 mmol) was dissolved in absolute EtOH (4 mL). A mixture of diethyl oxalate (310 mg, 2.1 mmol) and 1-(2-hydroxy-6-methanoxyphenyl)ethane-1-one (100 mg, 0.6 mmol) in absolute EtOH (2 mL) was slowly added to the NaOEt solution. The solution was reflux-stirred for 2 hours until complete consumption of starting material. The mixture was then allowed to cool to room temperature and neutralized with acq. HCl (2 M). The mixture was extracted with EtOAc (3 x 5 mL), the collected organic layer was dried over Na₂SO₄, filtered and concentrated under vacuum. Purification by silica (elution by gradient from 100:0 to 75:25 Cyclohexane/EtOAc) gave the pure title compound **21** (120 mg, 79%). **Characterization:** UPLC/MS Rt: 1.65 min (gradient 1), MS (ESI) m/z 249.0, [M+H]⁺. [M+H]⁺ calculated for C₁₇H₁₅O₅: 249.3. ¹H NMR (400 MHz, CDCl₃) δ 7.60 (t, *J* = 8.4 Hz, 1H), 7.16 (dd, *J* = 8.5, 0.9 Hz, 1H), 7.00 (s, 1H), 6.83 (d, *J* = 8.4 Hz, 1H), 4.44 (q, *J* = 7.1 Hz, 2H), 3.99 (s, 3H), 1.42 (t, *J* = 7.1 Hz, 3H).

### Step 2. Synthesis of 5-methoxy-4-oxo-4H-chromen-2-carboxylic acid (compound 22, Scheme 6).

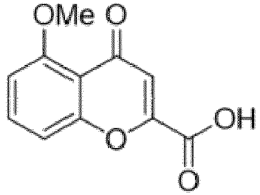

K₂CO₃ (100 mg, 0.60 mmol) was added to a solution of intermediate **21** (100 mg, 0.40 mmol) in a 3:1 mixture of THF/EtOH (4 mL). The reaction mixture was stirred for 6 h at 50°C until complete consumption of the starting material, then acq. HCl (2 M) was added up to reaching pH=5, and the mixture was extracted with EtOAc (3x4 mL). The collected organic layers were dried over Na₂SO₄, filtered and concentrated under vacuum to give the pure compound **22** (70 mg, 79% yield). **Characterization:** UPLC/MS Rt: 0.80 min (gradient 1), MS (ESI) m/z 220.9, [M+H]⁺. [M+H]⁺ calculated for C₁₁H₉O₅: 221.2. ¹H NMR (400 MHz, DMSO-d₆) δ 7.72 (t, *J* = 8.4 Hz, 1H), 7.18 (d, *J* = 8.4 Hz, 1H), 7.02 (d, *J* = 8.3 Hz, 1H), 3.86 (s, 3H).

### Step 3. Synthesis of N-(3,4-dimethoxyphenyl)-5-methoxy-4-oxo-4H-chromen-2-carboxamide (compound 23a, Scheme 6).

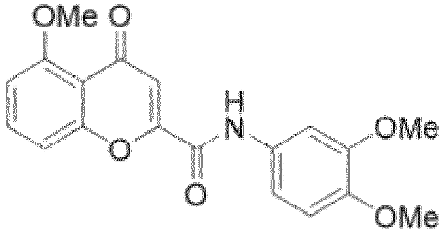

HATU (170 mg, 0.43 mmol) and DIPEA (0.23 mL, 1.3 mmol) were sequentially added to a solution of intermediate **22** (60 mg, 0.29 mmol) in a 3:1 mixture of DMF/DCM (4 mL) under argon. The reaction mixture was stirred at room temperature for 15 minutes, after that 3,4-dimethoxyaniline (44 mg, 0.29 mmol) was added and the reaction mixture was stirred for another 4 hours until complete consumption of the starting material. Water (1 mL) and acq. HCl (2 M) were added to reach pH=7, the mixture was extracted with CH₂Cl₂ (3x3 mL), the collected organic layers were dried over Na₂SO₄, filtered and concentrated under vacuum. Purification by silica (elution by gradient from 100:0 to 55:45 DCM/EtOAc) gave the pure title compound **23a** (92 mg, 90%).

**Characterization:** UPLC/MS Rt: 1.66 min (gradient 1), MS (ESI) m/z 356.0, [M+H]⁺. [M+H]⁺ calculated for C₁₉H₁₈NO₆: 356.3. ¹H NMR (400 MHz, DMSO-d₆) δ 7.78 (t, *J* = 8.4 Hz, 1H), 7.44 (d, *J* = 2.4 Hz, 1H), 7.36 (dd, J = 8.6, 2.4 Hz, 1H), 7.34 (d, J = 8.3 Hz, 1H), 7.05 (d, *J* = 8.3 Hz, 1H), 6.98 (d, *J* = 8.7 Hz, 1H), 6.76 (s, 1H), 3.88 (s, 3H), 3.77 (s, 3H), 3.76 (s, 3H).

### Step 4. Synthesis of N-(3,4-dihydroxyphenyl)-5-hydroxy-4-oxo-4H-chromen-2-carboxamide (compound 24a, Scheme 6).

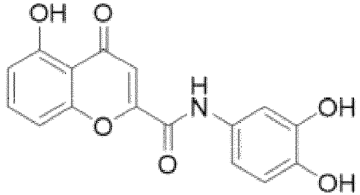

Compound **24a** was prepared following the general procedure 3, method B using: intermediate **23a** (45 mg, 0.13 mmol), BBrs (1 M in DCM) (0.59 mL, 0.59 mmol) in anhydrous CH₂Cl₂ (2.6 mL). The crude product was purified by silica (elution by gradient from 100:0 to 98:2 DCM/MeOH) to give product **24a** (0.016 g, 40% yield). **Characterization:** UPLC/MS Rt: 1.65 min (gradient 1), m/z 312.0, [M-H]⁻. [M-H]⁻ calculated for C₁₆H₁₀NO₆: 312.3. ¹H NMR (400 MHz, DMSO-d₆) δ 12.30 (bs, 1H), 10.48 (bs, 1H), 9.01 (bs, 1H), 7.77 (t, *J* = 8.4, Hz, 1H), 7.30 (d, *J* = 2.5 Hz, 1H), 7.26 (d, *J* = 8.4 Hz, 1H), 7.03 (dd, *J* = 8.5, 2.5 Hz, 1H), 6.95 (s, 1H), 6.88 (d, *J* = 8.3 Hz, 1H), 6.74 (d, *J* = 8.5 Hz, 1H).

### Example 14

### Synthesis of 3,4-dihydroxyphenyl-5-hydroxy-4-oxo-4H-chromen-2-carboxylate (compound 24b, Scheme 6)

Steps 1 to 2 of Example 13 are conducted followed by the further steps below.

### Step 3: Synthesis of 3,4-dimethoxyphenyl-5-methoxy-4-oxo-4H-chromen-2-carboxylate (compound 23b, Scheme 6)

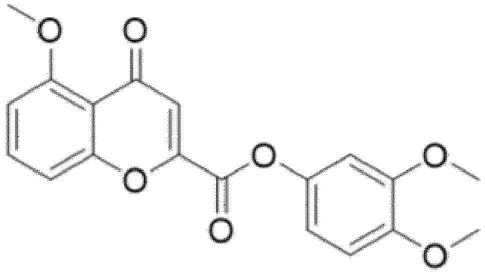

To a solution of 3,4-dimethoxyphenol (44 mg, 0.286 mmol) in anhydrous DCM (0.5 mL) at 0 °C intermediate **22** (63 mg, 0.286 mmol), DCC (59 mg, 0.286 mmol DMAP (3.5 mg, 0.0286 mmol) were added. After the disappearance of the starting material, the reaction mixture was filtered to remove the solid present and the crude material obtained was purified on silica gel (elution with gradient from 100:0 to 70:30 dichloromethane/AcOEt) to obtain a dirty yellow solid which was reprecipitated in a DCM/pentane mixture (3 mL/25 mL) to give, after filtration, product **23b** (25 mg, with 25% yield). **Characterization:** UPLC/MS Rt: 1.76 min (gradient 1), MS (ESI) m/z 357.0, [M+H]⁺. [M+H]⁺ calculated for C₁₉H₁₇O₇: 357.1. ¹H NMR (400 MHz, DMSO-d₆) δ 7.78 (t, J = 8.4 Hz, 1H), 7.26 (dd, J = 8.5, 0.9 Hz, 1H), 7.08 (d, J = 8.3 Hz, 1H), 7.05 - 7.00 (m, 2H), 6.96 (s, 1H), 6.87 (dd, J = 8.7, 2.7 Hz, 1H), 3.90 (s, 3H), 3.78 (s, 3H), 3.76 (s, 3H) .

### Step 4: Synthesis of 3,4-dihydroxyphenyl-5-hydroxy-4-oxo-4H-chromen-2-carboxylate (compound 24b, Scheme 6)

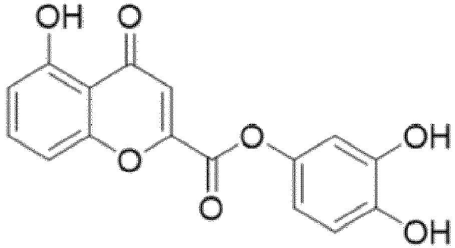

Compound **24b** was prepared according to general procedure 3 method B using: intermediate **23b** (22 mg, 0.062 mmol), BBrs (1 M in DCM) (0.28 mL, 0.28 mmol) in anhydrous CH₂Cl₂ (1.3 mL). The crude product was purified by crystallization (AcOEt/Pentane) to give product **24b** (15 mg, 77% yield) . **Characterization:** UPLC/MS Rt: 1.74 min (gradient 1), MS (ESI) m/z 313.1, [M-H]⁻. [M-H]⁻ calculated for C₁₆H₉O₇: 313.0. ¹H NMR (400 MHz, DMSO-d₆) δ 12.17 (s, 1H), 9.35 (s, 1H), 9.10 (s, 1H), 7.77 (t, J = 8.4 Hz, 1H), 7.23 (dd, J = 8.6, 0.9 Hz, 1H), 7.16 (s, 1H), 6.91 (dd, J = 8.3, 0.8 Hz, 1H), 6.78 (d, J = 8.6 Hz, 1H), 6.72 (d, J = 2.8 Hz, 1H), 6.58 (dd, J = 8.6, 2.8 Hz, 1H).

### Example 15

### Synthesis of 3,4-dihydroxyphenethyl-5-hydroxy-4-oxo-4H-chromen-2-carboxylate (compound 24c, Scheme 6)

Steps 1 to 2 of Example 12 are conducted followed by the further steps below.

### Step 3: Synthesis of 3,4-dimethoxyphenyl-5-methoxy-4-oxo-4H-chromen-2-carboxylate (compound 23c, Scheme 6)

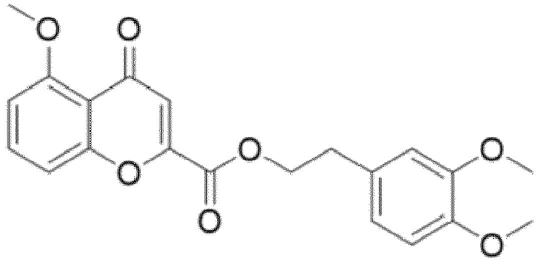

To a solution of 2-(3,4-dimethoxyphenyl)ethane-1-ol (66 mg, 0.363 mmol), in anhydrous DCM (0.6 mL), intermediate **22** (80 mg, 0.363 mmol), DCC (75 mg, 0.363 mmol), DMAP (4.4 mg, 0.0363 mmol) were added at a temperature of 0°C. After the disappearance of the starting material, the reaction mixture was filtered to remove the solid present and the crude material obtained was purified on silica gel (elution by gradient from 90:10 to 10:90 cyclohexane/AcOEt) to obtain product **23c** (62 mg, with 44% yield). **Characterization:** UPLC/MS Rt: 1.84 min (gradient 1), MS (ESI) m/z 385.1, [M+H]⁺.

[M+H]⁺ calculated for C₂₁H₂₁O₇: 385.1. ¹H NMR (400 MHz, DMSO-d₆) δ 7.74 (t, J = 8.4 Hz, 1H), 7.17 (dd, J = 8.5, 0.9 Hz, 1H), 7.04 (d, J = 8.3 Hz, 1H), 6.93 (d, J = 2.0 Hz, 1H), 6.88 (d, J = 8.2 Hz, 1H), 6.82 (dd, J = 8.1, 1.9 Hz, 1H), 6.71 (s, 1H), 4.49 (t, J = 6.7 Hz, 2H), 3.86 (s, 3H), 3.73 (s, 3H), 3.71 (s, 3H), 2.96 (t, J = 6.7 Hz, 2H).

### Step 4: Synthesis of 3,4-dihydroxyphenethyl-5-hydroxy-4-oxo-4H-chromen-2-carboxylate (compound 24c, Scheme 6)

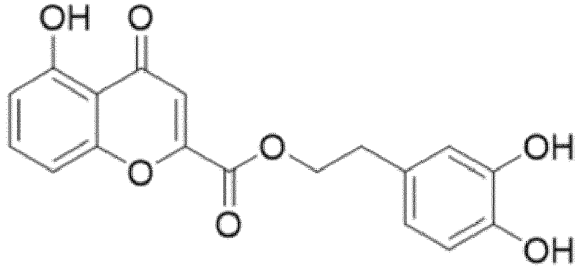

Compound **24c** was prepared according to general procedure 3 method B using: intermediate **23c** (60 mg, 0.156 mmol), BBrs (1 M in DCM) (0.47 mL, 0.47 mmol) in anhydrous CH₂Cl₂ (3.1 mL). The crude obtained was triturated with MeOH and the resulting yellow solid was filtered to give product **24c** (45 mg, 84% yield). **Characterization:** UPLC/MS Rt: 1.82 min (gradient 1), MS (ESI) m/z 341.3, [M-H]⁻. [M-H]⁻calculated for C₁₈H₁₃O₇: 341.1. ¹H NMR (400 MHz, DMSO-d₆) δ 12.14 (s, 1H), 8.80 (s, 1H), 8.74 (s, 1H), 7.75 (t, J = 8.4 Hz, 1H), 7.16 (dd, J = 8.5, 0.8 Hz, 1H), 6.92 (s, 1H), 6.89 (dd, J = 8.3, 0.8 Hz, 1H), 6.69 (d, J = 2.1 Hz, 1H), 6.66 (d, J = 8.0 Hz, 1H), 6.55 (dd, J = 8.0, 2.1 Hz, 1H), 4.45 (t, J = 6.9 Hz, 2H), 2.86 (t, J = 6.8 Hz, 2H).

### EXAMPLE 16

### Synthesis of N-(2-(3,4-dihydroxybenzamide)ethyl)-5-hydroxy-4-oxo-4H-chromen-2-carboxamide (Compound 28. Scheme 7).

### Step 1: Synthesis of tert-butyl (2-(5-methoxy-4-oxo-4H-chromen-2-carboxamide)ethyl)carbamate (compound 25, Scheme 7)

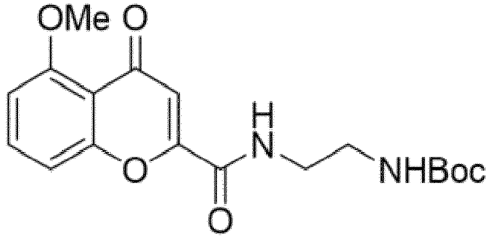

DIPEA (0.52 mL, 3 mmol), PyBOP (780 mg, 1.5 mmol), N-Boc-ethylenediamine (0.24 mL, 1.5 mmol) were sequentially added to a suspension of compound **22** (220 mg, 1 mmol) in a 4:1 mixture of DCM/DMF (10 mL) under argon atmosphere at 0°C. The reaction mixture was allowed to go to room temperature. After 30 minutes the conversion was complete. The resulting precipitate was filtered, the stock liquid was taken up with water and DCM and the aqueous phase was extracted with DCM (5 mL x 2), the combined organic phases were dried out with magnesium sulfate, filtered and concentrated under vacuum. The product was purified by chromatography on silica (elution by gradient from 100/0 to 94/6 DCM/MeOH) obtaining the pure intermediate **25** (252 mg, 79% yield). **Characterization:** UPLC/MS Rt: 1.52 min (gradient 1), MS (ESI) m/z: 363.1 [M+H]⁺. [M+H]⁺ Calculated for C₁₈H₂₃N₂O₆: 363.4. ¹H NMR (400 MHz, CDCl₃) δ 8.07 (s, 1H), 8.01 (s, 1H), 7.59 (t, *J* = 8.4 Hz, 1H), 7.13 (d, *J* = 8.5 Hz, 1H), 7.01 (s, 1H), 6.83 (dd, *J* = 8.4, 0.9 Hz, 1H), 3.97 (s, 3H), 3.56 (q, *J* = 5.2 Hz, 2H), 3.42 (m, 2H), 1.43 (s, 11H).

### Step 2: Synthesis of N-(2-aminoethyl)-5-methoxy-4-oxo-4H-chromen-2-carboxamide hydrochloride (compound 26, Scheme 7).

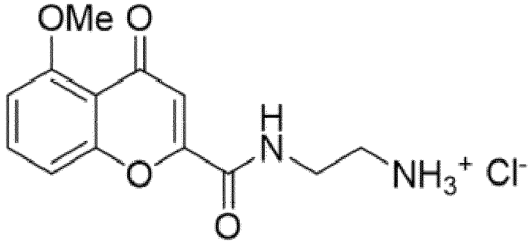

HCl (4 M in 1,4 dioxane) (0.7 mL, 2.82 mmol) was added dropwise to a solution of intermediate **25** (170 mg, 0.46 mmol) in anhydrous 1,4-dioxane (2.8 mL) under argon atmosphere. The conversion was complete after 2 hours, then the solvent was removed under vacuum. Trituration with Et₂O (1 mL) gave the desired intermediate **26** (122 mg, 87% yield). **Characterization:** UPLC/MS Rt: 1.45 min (gradient 1), MS (ESI) m/z: 263.1 [M+H]⁺.[M+H]⁺ Calculated for C₁₃H₁₅N₂O₄: 263.3. ¹H NMR (400 MHz, DMSO-*d₆*) δ 9.31 (t, *J* = 5.8 Hz, 1H), 8.08 (s, 3H), 7.76 (t, *J* = 8.4 Hz, 1H), 7.29 (dd, *J* = 8.5, 0.9 Hz, 1H), 7.03 (dd, *J* = 8.4, 1.0 Hz, 1H), 6.67 (s, 1H), 3.87 (s, 3H), 3.56 (q, *J* = 6.0 Hz, 2H), 3.02 (q, *J* = 5.9 Hz, 2H).

### Step 3: Synthesis of N-(2-(3,4-dimethoxybenzamido)ethyl)-5-methoxy-4-oxo-4H-chromen-2-carboxamide (compound 27, Scheme 7).

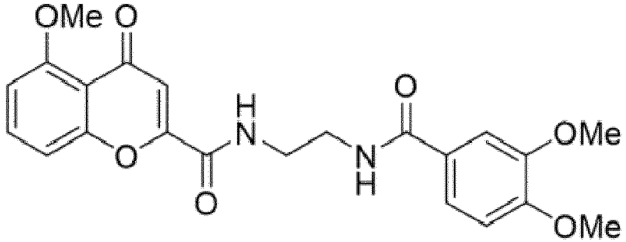

DIPEA (0.11 mL, 0.6 mmol) and PyBOP (157 mg, 0.30 mmol) were added to a solution of 3,4-dimethoxy benzoic acid (0.24 mL, 0.20 mmol) in a 4:1 mixture of DCM/DMF (3 mL) under argon atmosphere at 0°C. After 5 minutes, a solution of compound **26** in a 1:1 mixture of DIPEA/DMF (0.22 mL) was added dropwise to the reaction mixture under argon. The reaction mixture was allowed to go to room temperature. After 30 minutes the conversion was complete. The brine was added to the reaction mixture and the aqueous phase was extracted with DCM (5 mL x 2), the combined organic phases were dried out with sodium sulfate, filtered and concentrated under vacuum. The product was purified by chromatography on silica (elution by gradient from 100/0 to 94/6 DCM/MeOH) obtaining the pure intermediate **27** (57 mg, 66% yield). **Characterization:** UPLC/MS Rt: 1.37 min (gradient 1), MS (ESI) m/z: 425.3 [M-H]⁻.[M-H]- Calculated for C₂₂H₂₁N₂O₇: 425.4 ¹H NMR (400 MHz, DMSO-*d₆*) δ 9.20 (t, *J* = 5.6 Hz, 1H), 8.52 (t, *J* = 5.6 Hz, 1H), 7.75 (t, *J* = 8.4 Hz, 1H), 7.47 (dd, *J* = 8.3, 2.1 Hz, 1H), 7.44 (d, *J* = 2.1 Hz, 1H), 7.22 (d, *J* = 8.5 Hz, 1H), 7.03 (d, *J* = 8.4 Hz, 1H), 7.03 (d, *J* = 8.4 Hz, 1H), 6.63 (s, 1H), 3.86 (s, 3H), 3.80 (s, 3H), 3.78 (s, 3H), 3.49 - 3.40 (m, 4H).

### Step 3: Synthesis of N-(2-(3,4-dihydroxybenzamide)ethyl)-5-hydroxy-4-oxo-4H-chromen-2-carboxamide (Compound 28). Scheme 7).

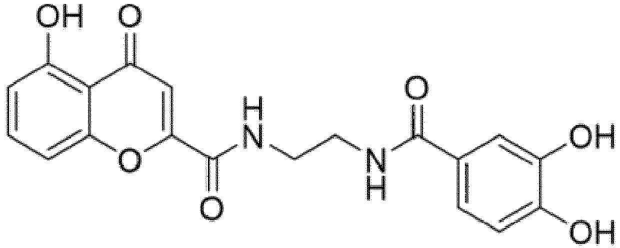

Compound **28** was prepared following the general procedure 3 method B using: intermediate **27 (27** mg, 0.06 mmol), BBrs (1 M in DCM) (0.27 mL, 0.27 mmol) in anhydrous DCM (3 mL). The crude was purified by trituration with MeOH (1 mL) obtaining the pure compound **28** (19 mg, 78%). **Characterization:** UPLC/MS Rt: 2.30 min (gradient 3), MS (ESI) m/z: 383.4 [M-H]⁻.[M-H]⁻ Calculated for C₁₉H₁₅N₂O₇: 383.3. ¹H NMR (400 MHz, DMSO-*d₆*) δ 9.20 (t, *J* = 5.6 Hz, 1H), 8.52 (t, *J* = 5.6 Hz, 1H), 7.75 (t, *J* = 8.4 Hz, 1H), 7.47 (dd, *J* = 8.3, 2.1 Hz, 1H), 7.44 (d, *J* = 2.1 Hz, 1H), 7.22 (d, *J* = 8.5 Hz, 1H), 7.03 (d, *J* = 8.4 Hz, 1H), 7.03 (d, *J* = 8.4 Hz, 1H), 6.63 (s, 1H), 3.86 (s, 3H), 3.80 (s, 3H), 3.78 (s, 3H), 3.49 - 3.40 (m, 4H).

### Example 17

### Synthesis of N-(2-(3-(3,4-dihydroxyphenyl)thiourea)ethyl)-5-hydroxy-4-oxo-4H-chromen-2-carboxamide (Compound 31. Scheme 7).

Steps 1 to 2 of Example 16 are conducted followed by the further steps below.

### Step 3: Synthesis of 4-isothiocyanate-1,2-dimethoxybenzene (Compound 29, Scheme 7).

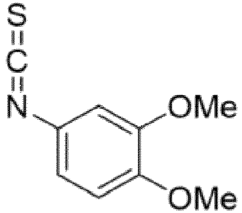

A solution of 3,4-dimethoxyaniline (300 mg, 1.95 mmol) in anhydrous CHCl₃ (2 mL) was added dropwise to a solution of TCDI (384 mg, 2.15 mmol) in anhydrous CHCl₃ (4 mL) under an inert atmosphere. The reaction mixture was stirred for one hour at 40°C. Then, the solvent was removed under vacuum and the crude was purified by chromatography on silica (elution by gradient from 100 to 85/15 cyclohexane/EtOAc) obtaining compound **29** (216 mg, 42% yield). **Characterization:** UPLC/MS Rt: 2.14 min (gradient 1), no ionization. ¹H NMR (400 MHz, CDCl₃) δ 6.83 (dd, *J* = 8.6, 2.2 Hz, 1H), 6.78 (d, *J* = 8.5 Hz, 1H), 6.74 (d, *J* = 2.2 Hz, 1H), 3.88 (s, 3H), 3.87 (s, 3H) .

### Step 4: Synthesis of N-(2-(3-(3,4-dimethoxyphenyl)thiourea)ethyl)-5-methoxy-4-oxo-4H-chromen-2-carboxamide (Compound 30, Scheme 7).

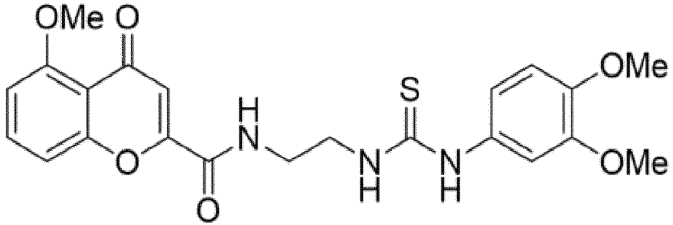

DIPEA (0.18 mL, 1.00 mmol) and a solution of compound **29** (39 mg, 0.20 mmol) in EtOH (1 mL) were sequentially added to a solution of compound **26** (60 mg, 0.20 mmol) in EtOH (1 mL) under argon. The reaction mixture was reflux-stirred for 15 minutes. Then, the solvent was removed under vacuum. The crude was purified by trituration with an 8:2 mixture of DCM/MeOH (1 mL) obtaining the pure intermediate **30** (44 mg, 49% yield). **Characterization:** UPLC/MS Rt: 1.44 min (gradient 1), MS (ESI) m/z: 458.1 [M+H]⁺.[M+H]⁺ Calculated for C₂₂H₂₄N₃O₆S: 458.5. ¹H NMR (400 MHz, DMSO-*d₆*) δ 9.46 (s, 1H), 9.14 (t, *J* = 5.6 Hz, 1H), 7.76 (t, *J* = 8.4 Hz, 1H), 7.64 (s, 1H), 7.23 (d, *J* = 8.5 Hz, 1H), 7.03 (d, *J* = 8.3 Hz, 1H), 6.95 (d, *J* = 2.4 Hz, 1H), 6.86 (d, *J* = 8.6 Hz, 1H), 6.77 (dd, *J* = 8.5, 2.4 Hz, 1H), 6.63 (s, 1H), 3.87 (s, 3H), 3.70 (s, 3H), 3.68 (m, 2H), 3.65 (s, 3H), 3.49 (q, *J* = 5.7 Hz, 2H).

### Step 5: Synthesis of N-(2-(3-(3,4-dihydroxyphenyl)thiourea)ethyl)-5-hydroxy-4-oxo-4H-chromen-2-carboxamide (Compound 31, Scheme 7).

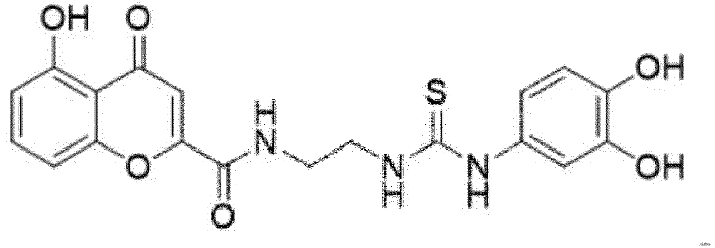

Compound **31** was prepared following the general procedure 3 method B using: intermediate **30** (40 mg, 0.09 mmol), BBrs (1 M in DCM) (0.41 mL, 0.41 mmol) in anhydrous DCM (4 mL). The crude was purified by chromatography on silica (elution by gradient from 100 to 92/8 CHCl₃/EtOH) obtaining the non-pure compound. Then the compound eluted by chromatography was triturated with a 1:1 cyclohexane/EtOAc mixture (1 mL) obtaining the pure compound **31** (12 mg, 31% yield). **Characterization:** UPLC/MS Rt: 1.48 min (gradient 1), MS (ESI) m/z: 414.3 [M-H]⁻.[M-H]⁻ Calculated for C₁₉H₁₆N₃O₆S : 414.4. ¹H NMR ¹H NMR (400 MHz, MeOD) δ 7.68 (t, *J* = 8.4 Hz, 1H), 7.16 (d, J = 8.4 Hz, 1H), 6.94 (s, 1H), 6.84 (d, *J* = 8.3 Hz, 1H), 6.74 (d, *J* = 8.3 Hz, 1H), 6.67 (d, *J* = 2.4 Hz, 1H), 6.56 (dd, *J* = 8.4, 2.5 Hz, 1H), 3.85 (t, J = 5.6 Hz, 2H), 3.60 (t, J = 6.1 Hz, 2H) .

### Example 18

### Enzymatic activity

The activity of the compounds of the invention was assayed experimentally by evaluating the enzymatic activity of RNA-dependent RNA polymerase (SARS-CoV-2) using an *in vitro* enzymatic assay.

The RdRp reactions were conducted in duplicate at 37°C for 60 minutes in a 10 µl mixture containing assay buffer, duplex RNA, substrate and ATP enzyme and a compound according to the invention. The 10 µl reactions were conducted in 384-well wells of Optiplate (PerkinElmer) .

The dilutions of the compounds (starting from a 10 mM stock solution in DMSO of the substrate) were prepared in analysis Tris-buffer pH 8.0 containing some amount of detergent to prevent non-specific aggregation (5% DMSO concentration) and 2 µl of the dilution was added to 6 µl of RdRp containing RNAse inhibitor for preincubation (30 minutes at room temperature with slow stirring). The reaction was initiated by the addition of 2 µl of the substrate mixture containing duplex RNA and ATP substrate. The final DMSO concentration was 1% in all the reactions (reference compound - 0% DMSO).

After the enzymatic reactions, 10 µl of anti-Dig Acceptor microspheres (PerkinElmer, diluted 1:500 with 1x detection buffer) were added to the reaction mixture. After brief stirring, the plate was incubated for 30 minutes.

Finally, 10 µl Streptavidin AlphaScreen-conjugated donor beads (Perkin, diluted 1:125 with 1x detection buffer) were added.

In 30 minutes, the samples were measured in the AlphaScreen microplate reader (EnSpire Alpha 2390 Multilabel Reader, PerkinElmer) .

The IC₅₀ data and the percentage inhibition of the enzyme at the 25 and 100 µM concentration of the compounds against RdRp are summarized in the following Table:

| Ref | Formula | IC50 (µM) | % inhib. (25 µM) | % inhib. (100 µM) |
|---|---|---|---|---|
| **(5a)** | 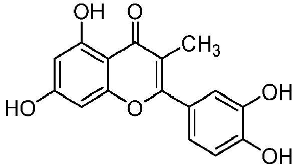 | 4.3 ± 1.1 | 94 | 99 |
| **(5b)** | 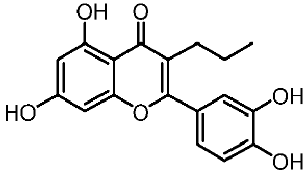 | 8.8 ± 0.7 | 72 | 98 |
| **(5c)** | 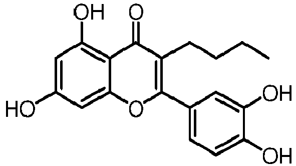 | 6.0 ± 1.1 | 81 | 100 |
| **(9a)** | 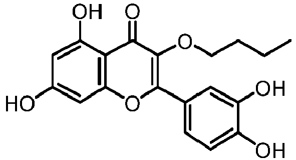 | 5.3 ± 1.0 | 52 | 98 |
| **(9b)** | 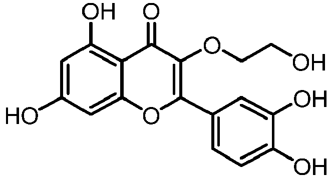 | 6.1 ± 1.0 | 83 | 100 |
| **(9c)** | 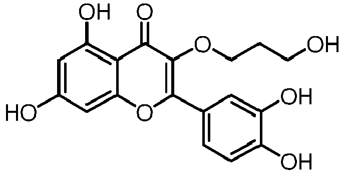 | 5.6 ± 0.1 | 71 | 95 |
| **(13a)** | 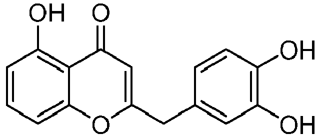 | 8.5 ± 0.7 | 78 | 99 |
| **(13b)** | 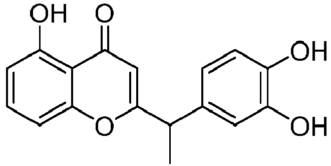 | 4.1 ± 1.4 | 65 | 100 |
| **(13c)** | 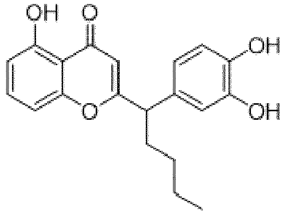 | > 100 | 16 | 37 |
| **(13d)** | 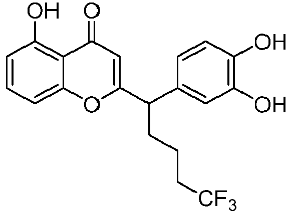 | NO inhibition | 8 | 10 |
| **(18)** | 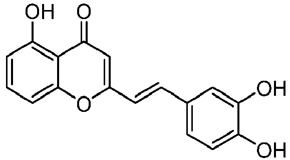 | 7.0 ± 1.2 | 54 | 94 |
| **(20)** | 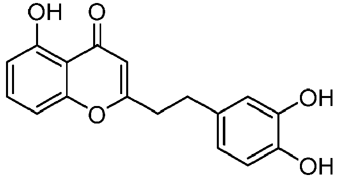 | 5.8 ± 0.7 | 80 | 97 |
| **(24a)** | 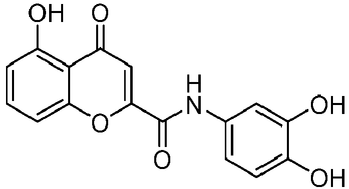 | 7.1 ± 0.8 | 80 | 100 |
| **(24b)** | 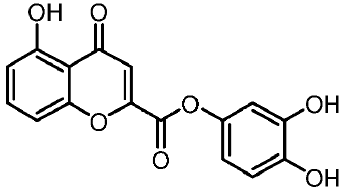 | 2.0 ± 1.0 | 95 | 94 |
| **(24c)** | 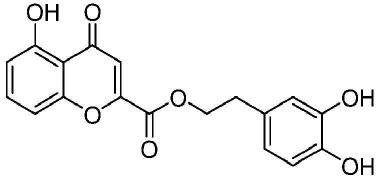 | 44.1 ± 1.1 | 36 | 72 |
| **(28)** | 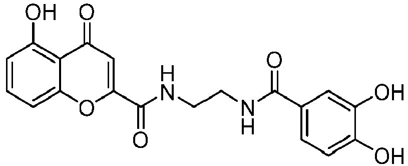 | 15.9 ± 1.1 | 79 | 100 |
| **(31)** | 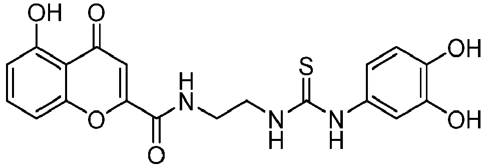 | 3.4 ± 1.2 | 86 | 94 |

For compounds **28** and **31,** the 10 mM stock solution in DMSO and subsequent dilutions were freshly prepared in order to avoid degradation of the compound, then revealed by NMR analysis.

### Example 19

### Solubility and stability

The solubility and stability of some compounds of the invention and known compounds were evaluated. The data are shown in the following Table.

| Compound | Kinetic solubility Sk (µM) | Plasma stability in mice t_{1/2} (minutes) | Microsomal stability in mice t_{1/2} (minutes) |
|---|---|---|---|
| luteolin | 21 | >120 | >60 |
| quercetin | 16 | 7 | >60 |
| **(5a)** | 32 | - | - |
| **(5b)** | 13 | - | - |
| **(5c)** | < 1 | - | - |
| **(9a)** | 156 | >120 | >60 |
| **(9b)** | 167 | >120 | >60 |
| **(9c)** | 231 | 90 | 60 |
| **(13a)** | 63 | >120 | >60 |
| **(13b)** | 165 | >120 | >60 |
| **(18)** | <1 | - | - |
| **(20)** | 67 | >120 | >60 |
| **(24a)** | 65 | 83 ± 13 | 60 |
| **(24b)** | <1 | - | - |
| **(31)** | <1 | - | - |

Compounds **9a, 9b, 9c, 13a, 13b, 20** and **24a** gave the best results in terms of plasma and microsomal solubility and stability.

### Example 20

### Control compound inhibition percentage

The inhibitory activity towards RdRp was evaluated for screening compounds similar to those of the invention and the results are shown in the following Table:

| Formula | % inhibition | |
|---|---|---|
| | 25 µM | 100 µM |
| 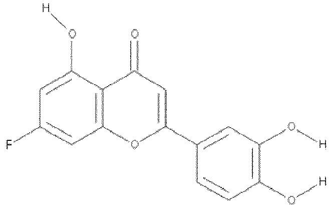 | 0 | 25 |
| 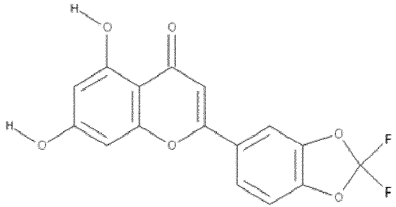 | 0 | 51 |
| 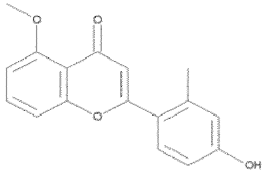 | 3 | 34 |

The above data demonstrate the poor inhibition activity of the tested compounds despite the structural similarity.

From the above, the advantages linked with the present invention will be immediately apparent to those skilled in the art.

Firstly, the compounds described are referred to as small molecules, thus molecules of small size capable of being easily synthesized.

The main formula and substructures define scaffolds which allow a good chemical diversity, allowing many compounds with similar structure to be investigated.

In addition to this, the compounds described have shown to be very active in inhibiting the replication of the virus responsible for the SARS-CoV-2 syndrome with IC₅₀ values in the order of a few micromoles.

The activity of the described compounds is not limited to the SARS-CoV-2 virus alone, but also comprises other RNA viruses, such as the *Hepatitis C* virus (HCV), Ebola virus and Zika virus.

It should be emphasized that the present invention contemplates already known compounds, and therefore belongs to the so-called *repurposing* of drugs, thus making the evaluations of activity and toxicity of a possible drug for the new identified disease faster.

## Claims

1. A compound having the following general formula (I): 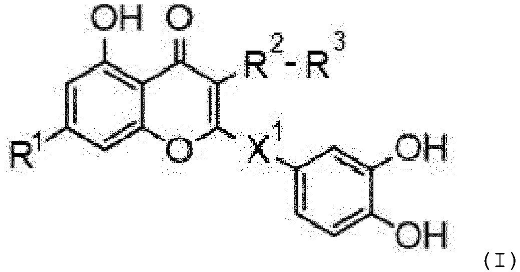 wherein
R¹ is selected from hydrogen or -OH, -N(R₄)R₅, -NO₂, halogen, C₁₋₄ haloalkyl, wherein
R₄ and R₅ are independently H or C₁₋₄ alkyl,
R² is selected from hydrogen, C₁₋₄ alkyl, C₁₋₄ haloalkyl, C₁₋₇ alkoxyalkyl, or
-O-, -NH-,
R³ is H, -OH, or is selected from C₁₋₄ alkyl, C₁₋₄ hydroxyalkyl, C₁₋₄ haloalkyl, C₁₋₄ aminoalkyl, C₁₋₄ alkoxyalkyl,
X¹ is X² and R¹ and R² are both hydrogen and said compounds have general formula (Ib): 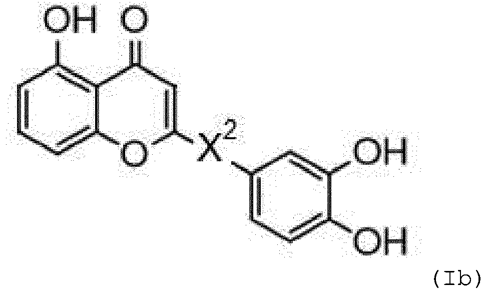
wherein X² is selected from -CH₂-, -CH₂₋CH₂-, -CH=CH-, -CHR⁴, - COR⁵,
-NH, -O- or -OR^{6,} where
R₄ is selected from C₁₋₄ alkyl, C₁₋₄ haloalkyl, C₁₋₄ alkoxyalkyl, C₁₋₄ aminoalkyl,
R⁵ is selected from -NH-, -O-, -NHR⁶-, -OR⁶-,
R⁶ is C₂₋₄ alkyl,
or
wherein X¹ is X³ and R¹ and R² are both hydrogen and said compounds have general formula (Ic): 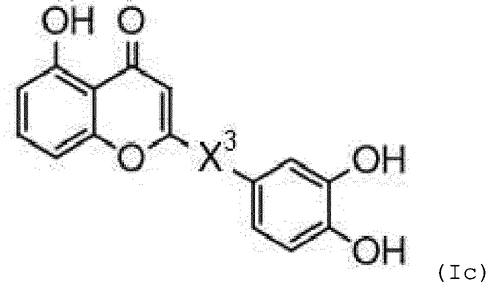
wherein X³ is selected from -C(O)X⁴ or -C(O)X⁴-(CH₂)₂₋₇-X⁵ where X⁴ is selected from -NH, -O- or -O-C₂-₄ alkyl,
X⁵ is selected from: 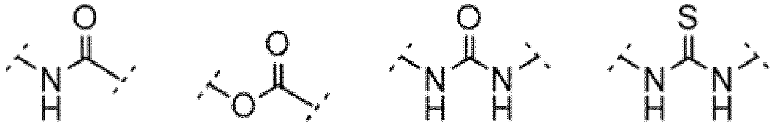 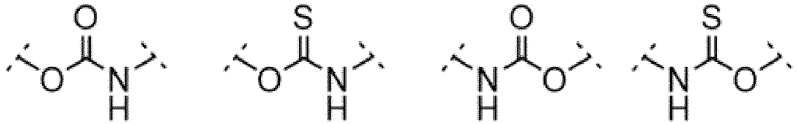 or
X⁵ is selected from -NH-R⁷, -O-R⁷ or -O-C₂-₄ alkyl-R⁷ where R⁷ is selected from -C(O), -C(O)NH or -C(S)NH, -C(O)O-, -C(S)O-, with the proviso that the following compound is excluded:

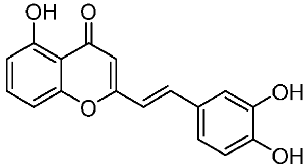

2. A compound according to claim 1, wherein in general formula (I) : 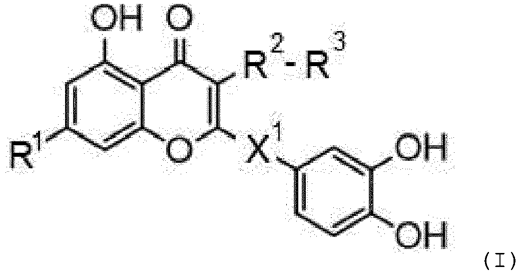 when R² is C₁₋₇ alkoxyalkyl, it is preferably C₁₋₄ alkoxyalkyl.

3. A compound according to claim 1, wherein said compound has the following formula (Ib) 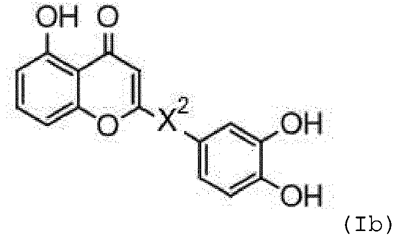 wherein
X² is preferably selected from -CH₂-, -CH₂CH₂-, -CH=CH-, -CHR⁴, -COR⁵ and -OR⁶, where
R⁴ is selected from C₁₋₄ alkyl, C₁₋₄ haloalkyl, C₁₋₄ alkoxyalkyl, C₁₋₄ aminoalkyl,
R⁵ is selected from -NH-, -O-, -NHR⁶-, -OR⁶-,
R⁶ can be C₂₋₄ alkyl.

4. A compound according to claim 1 having a formula selected from:

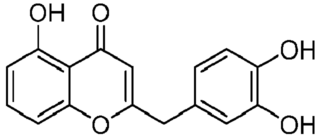

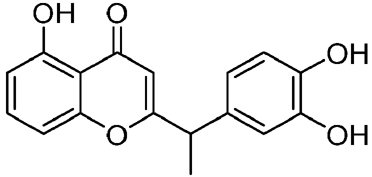

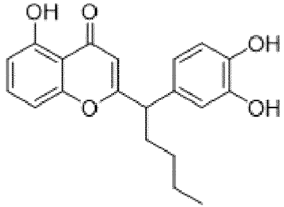

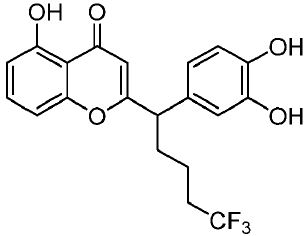

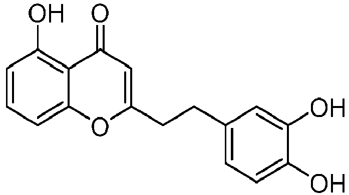

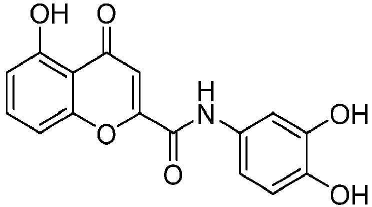

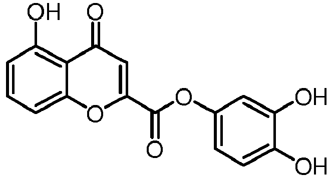

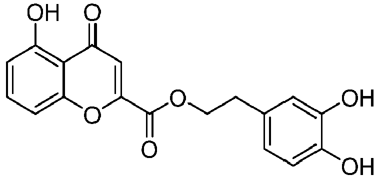

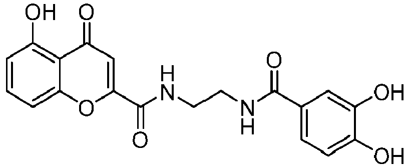

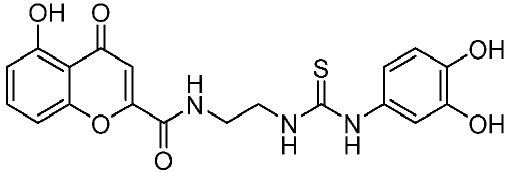

5. A compound having the following general formula (I): 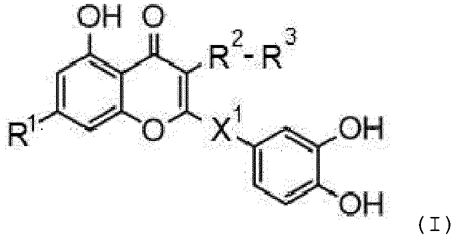 wherein
R¹ is selected from hydrogen or -OH, -N(R₄)R₅, -NO₂, halogen, C₁₋₄ haloalkyl, where
R₄ and R₅ are independently H or C₁₋₄ alkyl,
R² is selected from hydrogen, C₁₋₄ alkyl, C₁₋₄ haloalkyl, C₁₋₇ alkoxyalkyl, or -O-, -NH-,
R³ is H, -OH, or is selected from C₁₋₄ alkyl, C₁₋₄ hydroxyalkyl, C₁₋₄ haloalkyl, C₁₋₄ aminoalkyl, C₁₋₄ alkoxyalkyl,
X¹ is a single bond and R¹ is -OH and said compounds have general formula (Ia): 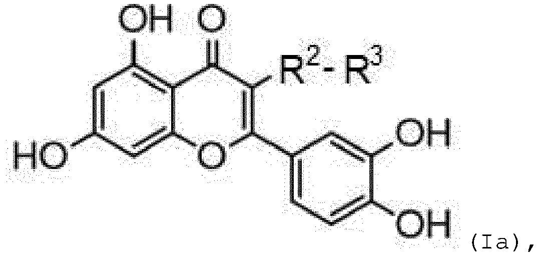 or
X¹ is X² and R¹ and R² are both hydrogen and said compounds have general formula (Ib): 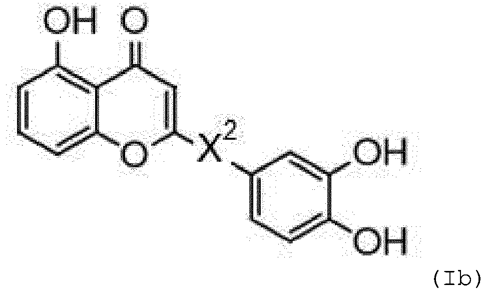
wherein X² is selected from -CH₂-, -CH₂₋CH₂-, -CH=CH-, -CHR⁴, -COR⁵, -NH, -O- or -OR^{6,} where
R⁴ is selected from C₁₋₄ alkyl, C₁₋₄ haloalkyl, C₁₋₄ alkoxyalkyl, C₁₋₄ aminoalkyl,
R⁵ is selected from -NH-, -O-, -NHR⁶-, -OR⁶-,
R⁶ is C₂₋₄ alkyl,
or
wherein X¹ is X³ and R¹ and R² are both hydrogen and said compounds have general formula (Ic): 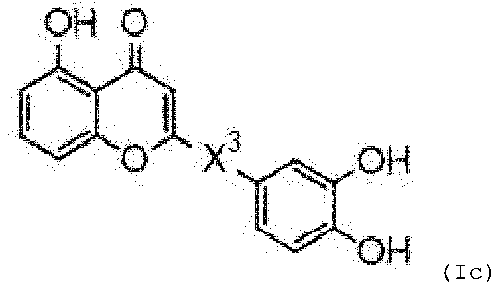
where X³ is selected from -C(O)X⁴ or -C(O)X⁴-(CH₂)₂₋₇-X⁵ and preferably -C(O)X⁴-(CH₂)₂₋₄-X⁵ where
X⁴ is selected from -NH, -O- or -O-C₂-₄ alkyl,
X⁵ is selected from: 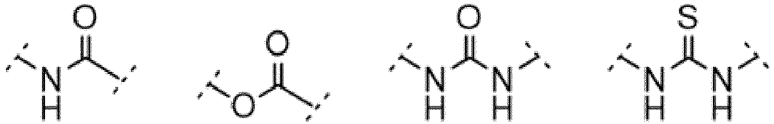 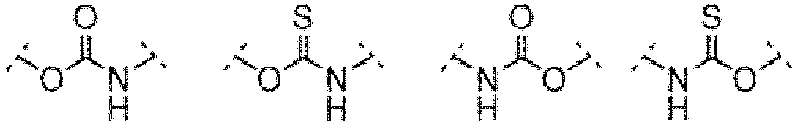 or
X⁵ is selected from -NH-R⁷, -O-R⁷ or -O-C₂-₄ alkyl-R⁷ where R⁷ is selected from -C(O), -C(O)NH or -C(S)NH, -C(O)O-, -C(S)O-for medical use.

6. A compound according to the preceding claim, for medical use in the prevention and/or treatment of a disease caused by an RNA virus.

7. A compound for medical use according to the preceding claim 5 or 6, wherein said RNA virus is selected in the group comprising: SARS-CoV-2 virus, Hepatitis C virus (HCV), Ebola virus, Zika virus and the further viruses: Severe acute respiratory syndrome coronavirus (SARS), Middle east respiratory syndrome coronavirus (MERS), influenza, Orthomyxoviruses, Paramyxoviruses, Hendra and Nipah viruses, Measles, Picornaviruses, Poliomyelitis ("Polio"), Hepatitis A virus (HAV), Rotavirus, Human immunodeficiency virus (HIV), adult Human T-cell lymphotropic virus type 1 (HTLV-1).

8. A compound for medical use according to any one of the preceding claims 5 to 7, wherein said RNA virus is represented by SARS-CoV-2.

9. A compound for medical use according to any one of the preceding claims 5 to 8, having general formula (I): 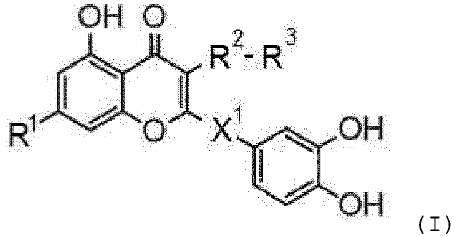 wherein
when R² is C₁₋₇ alkoxyalkyl, it is preferably C₁₋₄ alkoxyalkyl.

10. A compound for medical use according to any one of the preceding claims 5 to 8, having general formula (Ic): 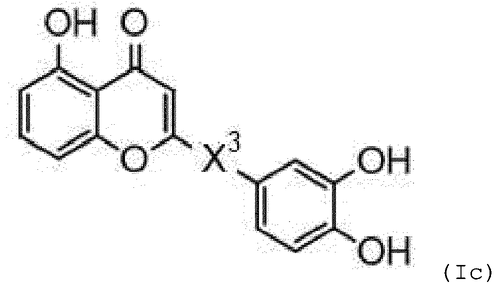 wherein when X³ is -C(O)X⁴-(CH₂)₂₋₇-X⁵, it is preferably -C(O)X⁴-(CH₂)₂₋₄-X⁵.

11. A compound for medical use according to any one of the preceding claims 5 to 8, wherein said compound has general formula 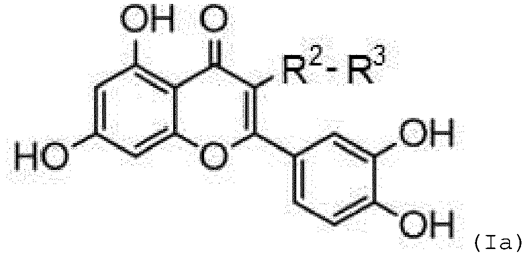 wherein R² is -O- and R³ is C₁₋₄ alkyl or C₁₋₄ hydroxyalkyl.

12. A compound for medical use according to any one of the preceding claims 5 to 8, wherein said compound has general formula 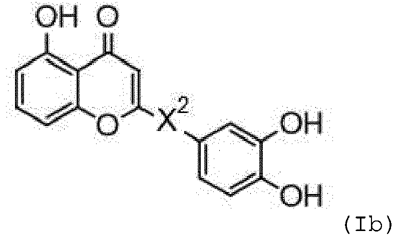 wherein
X² is preferably selected from -CH₂-, -CH₂CH₂-, -CH=CH-, -CHR⁴, -COR⁵ and -OR⁶, where
R⁴ is selected from C₁₋₄ alkyl, C₁₋₄ haloalkyl, C₁₋₄ alkoxyalkyl, C₁₋₄ aminoalkyl,
R⁵ is selected from -NH-, -O-, -NHR⁶-, -OR⁶-,
R⁶ is C₂₋₄ alkyl.

13. A compound for medical use according to any one of the preceding claims 5 to 12, having a formula selected from:

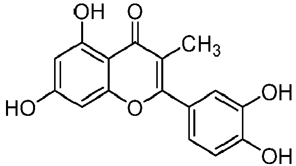

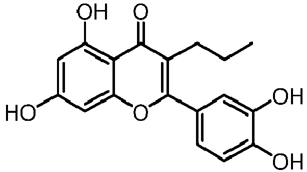

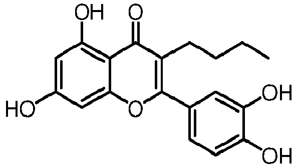

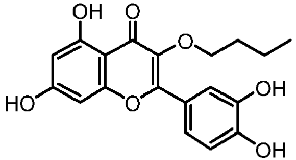

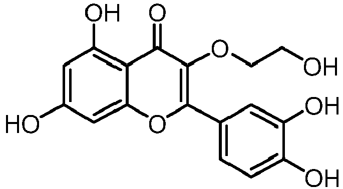

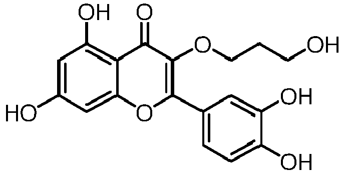

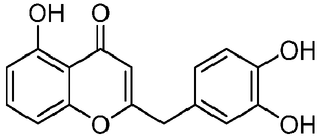

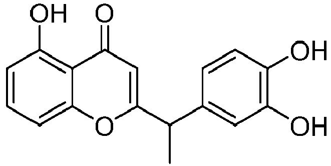

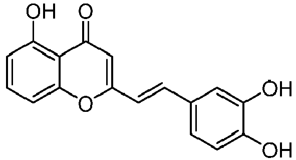

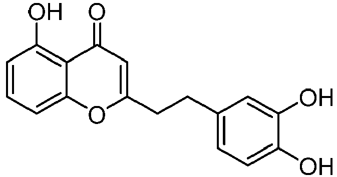

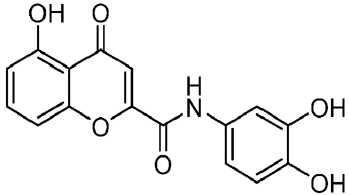

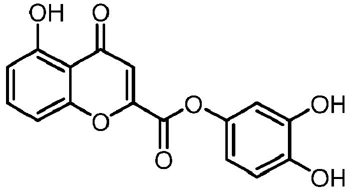

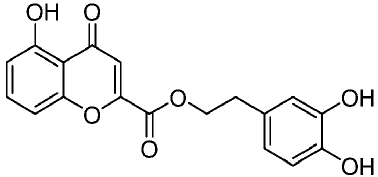

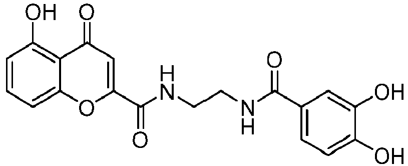

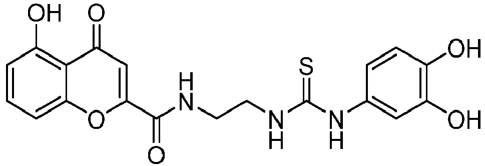

14. A compound for medical use according to any one of the preceding claims 5 to 13, having the formula selected from:

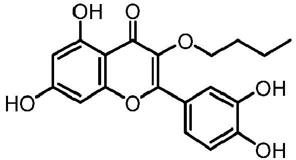

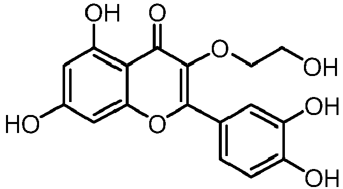

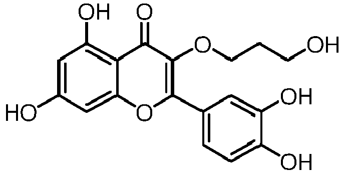

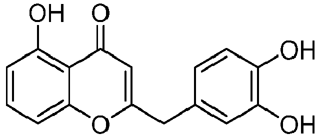

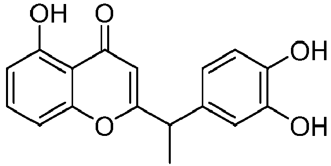

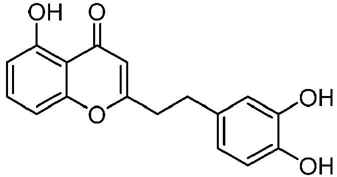

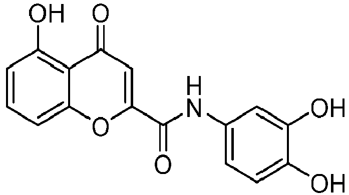

15. A pharmaceutical formulation comprising a compound according to any one of claims 1 to 4.
